(19) Europäisches Patentamt
European Patent Office
Office européen des brevets

(11) EP 0 815 153 B1

(12) **FASCICULE DE BREVET EUROPEEN**

(45) Date de publication et mention de la délivrance du brevet:
**04.05.2005 Bulletin 2005/18**

(21) Numéro de dépôt: **97907149.5**

(22) Date de dépôt: **28.02.1997**

(51) Int Cl.[7]: **C08G 18/70**, C07C 263/18, C08G 18/80, C07F 9/09, C07F 9/40, C07F 9/38, C09D 175/04

(86) Numéro de dépôt international:
**PCT/FR1997/000359**

(87) Numéro de publication internationale:
**WO 1997/031960 (04.09.1997 Gazette 1997/38)**

(54) **COMPOSITIONS A BASE D'ISOCYANATE, LEUR PROCEDE D'UTILISATION, LEUR UTILISATION POUR REALISER DES REVETEMENTS ET REVETEMENTS AINSI OBTENUS**

ISOCYANATZUSAMMENSETZUNGEN, VERFAHREN ZU DEREN VERWENDUNG, DEREN VERWENDUNG ZUR HERSTELLUNG VON BESCHICHTUNGEN UND BESCHICHTUNGEN SO ERHALTEN

ISOCYANATE COMPOSITIONS, METHOD FOR USING SAME, USE THEREOF FOR PRODUCING COATINGS, AND RESULTING COATINGS

(84) Etats contractants désignés:
**AT BE CH DE DK ES FI FR GB GR IE IT LI LU MC NL PT SE**

(30) Priorité: **29.02.1996 FR 9602710**

(43) Date de publication de la demande:
**07.01.1998 Bulletin 1998/02**

(60) Demande divisionnaire:
**05001315.0 / 1 524 281**

(73) Titulaire: **RHODIA CHIMIE**
**92408 Courbevoie Cédex (FR)**

(72) Inventeurs:
- **NABAVI, Minou**
  **F-75014 Paris (FR)**
- **JEANNETTE, Thierry**
  **F-92380 Garches (FR)**
- **LYOTHIER, Arnaud**
  **F-93300 Aubervilliers (FR)**
- **BERNARD, Jean-Marie**
  **F-69440 Mornant (FR)**

(74) Mandataire: **Jacobson, Claude et al**
**Cabinet Lavoix**
**2, Place d'Estienne d'Orves**
**75441 Paris Cedex 09 (FR)**

(56) Documents cités:
**EP-A- 0 004 927**
**EP-A- 0 367 667**
**EP-A- 0 510 438**
**WO-A-94/22935**
**DE-A- 3 108 537**
**FR-A- 1 278 286**
**FR-A- 2 437 292**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 815 153 B1

## Description

La présente invention a pour objet des composés et des compositions à base d'isocyanate (qui peuvent être partiellement masqués, mais cela n'est pas la mise en oeuvre préférée). Elle vise également leur procédé d'utilisation, leur utilisation pour réaliser des revêtements et revêtements ainsi obtenus. Elle concerne plus particulièrement les compositions (auto)dispersibies en phase aqueuse.

Pour mieux comprendre l'invention il a paru opportun de faire les rappels suivants.

Dans la présente description, les caractéristiques de granulométrie font souvent référence à des notations du type $d_n$ où n est un nombre de 1 à 99, cette notation est bien connue dans de nombreux domaines techniques, mais est un peu plus rare en chimie, aussi peut-il être utile d'en rappeler la signification. Cette notation représente la taille de particule telle que n % (en poids, ou plus exactement en masse, puisque le poids n'est pas une quantité de matière mais une force) des particules soit inférieur ou égal à ladite taille.

Dans la suite de la description, on utilisera l'indice de polydispersité, lequel est défini comme

$$I = (d_{90}-d_{10})/d_{50}$$

Dans l'activité des peintures et des vernis, on utilise largement les diisocyanates notamment les alcoylènes diisocyanates (par exemple ceux vendus sous la marque Tolonate) et leurs dérivés de type biuret ou leurs trimères.

Toutefois, deux problèmes restent à ce jour incomplètement résolus, à savoir :

- l'utilisation de solvant organique, dont la présence est réputée toxique et néfaste pour l'environnement ;
- la nécessité de mettre sur le marché des produits non volatils qui a conduit à alourdir les molécules et ce en oligomérisant les diisocyanates ; cette solution n'est pas entièrement satisfaisante car elle utilise une fonction élaborée, donc chère, pour résoudre le problème.

Bien entendu, ces problèmes doivent être résolus en respectant les contraintes intrinsèques aux revêtements.

Pour réaliser des films de peintures ou de vernis, on mélange d'une part une dispersion, ou une émulsion, contenant l'isocyanate qui peut être bloqué et d'autre part une dispersion ou une solution de polyol.

Le mélange des dispersions pouvant également contenir des pigments, et notamment du bioxyde de titane, dont la dispersion est améliorée par la présente invention, et des charges est alors déposé sur un support sous forme d'un film à l'aide des techniques classiques de mise en oeuvre des peintures industrielles. Lorsque la préparation contient des isocyanates bloqués, l'ensemble film plus support est cuit à une température suffisante pour assurer le déblocage des fonctions isocyanates et la condensation de celles-ci avec des groupements hydroxyles des particules de polyol. Il convient toutefois de rappeler que les produits masqués ou bloqués présentent un prix de revient significativement plus élevé que celui des produits non masqués.

L'utilisation de solvants organiques est de plus en plus souvent critiquée par les autorités en charge de la sécurité du travail, car ces solvants, ou du moins certains d'être eux, sont réputés toxiques ou chronotoxiques. C'est la raison pour laquelle on essaye de développer de plus en plus des techniques qui se substituent aux techniques en milieu solvant pour pallier les inconvénients afférents aux solvants.

Une des solutions les plus souvent utilisées réside dans l'utilisation d'émulsions ou de dispersions dans l'eau. En raison de la réactivité de l'eau avec les isocyanates, cette solution est surtout utilisée pour les isocyanates masqués.

Pour ne pas tomber de Charybde en Scylla, un écueil majeur est à éviter, à savoir détériorer une ou plusieurs des qualités essentielles des revêtements [caractère lisse (éviter la "peau d'orange"), dureté, résistance aux solvants,.....], en particulier, il faut craindre une mauvaise adhésion du revêtement sur son support. En effet beaucoup de tensioactifs sont réputés altérer la solidité du lien entre le revêtement et son support et sont connus et utilisés pour défavoriser l'accrochage entre un polymère et un support.(confère par exemple DE-OS 3.108.537).

Lorsque l'on utilise des isocyanates non ou incomplètement masqués, la durée pendant laquelle il sont utilisables reste inférieur à quelques heures, En général une ou deux heures.

Aussi est-il important de ne pas rencontrer de difficulté lors de la mise en émulsion ou en dispersion des isocyanates.

C'est pourquoi un des buts de la présente invention est de fournir une composition qui permette par mélange dans l'eau ou plus précisément dans une phase aqueuse, d'obtenir une émulsion sans qu'il soit pour cela nécessaire d'utiliser des techniques et/ou des installations spécifiques.

Un autre but de la présente invention est de fournir une composition du type précédent qui ne perturbe pas les opérations de revêtement.

Un autre but de la présente invention est de fournir une composition du type précédent dont la teneur en solvant soit inférieur à 1/5, avantageusement à 1/10, en masse de ladite composition. Bien sûr il est préférable qu'il

n'y en ait le moins possible, et même pas du tout.

Ces buts et d'autres qui apparaîtront par la suite sont atteints au moyen d'une composition à base d'isocyanate(s), avantageusement non masqué(s), où elle comporte au moins un composé comportant une fonction anionique et avantageusement un fragment de chaîne polyéthylène glycol d'au moins 1, de préférence d'au moins 5 unités éthylènyloxyles

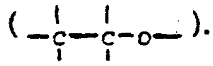

ainsi la présente invention vise pour addition successive ou simultanée, une composition selon la revendication 1.

Selon la présente invention, ledit composé peut-être utilisé seul, ou en mélange avec un ou plusieurs agents tensioactifs. Ces derniers peuvent être des agents répondant aussi à l'obligation ci-dessus de comporter une fonction anionique et avantageusement un fragment de chaîne polyéthylène glycol de préférence d'au moins 5 unités éthylènyloxyles.

Ces éventuels agents tensioactifs peuvent également être choisis parmi d'autres composés ioniques [notamment sulfate ou phosphate d'aryle(s) et/ou d'alcoyle(s) (bien entendu aryle englobe notamment les alcoylaryles et alcoyle englobe notamment les aralcoyles), les aryl- ou alcoyl--phosphonate, -phosphinate, sulfonate, sel d'acide gras et/ou zwitterioniquel et parmi les composés non-ioniques ceux bloqués en bout de chaîne ou non. (toutefois les composés non ioniques présentant des fonctions alcooliques sur au moins l'une des chaînes semblent avoir un effet légèrement défavorable sur l'(auto)émulsion même si ils ont un effet favorable sur d'autres aspects de la composition pour peinture ; compte tenu de cela il est préférable que la teneur en ce type de composé représentent au plus un tiers, avantageusement au plus un cinquième, de préférence au plus un dixième en masse desdits composés anioniques selon l'invention)

Avantageusement ledit composé comporte une partie hydrophile formée de ladite fonction anionique, dudit (éventuel) fragment de chaîne polyéthylène glycol et d'une partie lipophile à base d'un radical hydrocarboné.

Ladite partie lipophile est en général choisie parmi les groupes alcoyles [dans la présente description ALCO-*yle* est pris dans son sens étymologique de reste hydrocarboné d'un ALCO-*ol* après ignorance de la fonction alcool (ou ol)] ;et aryles. Lorsque le nombre de fonction éthylène glycol est au plus égale à 5, les alcoyles simples sont avantageusement ramifiés, avantageusement de $C_8$ à $C_{12}$, les aralcoyles $C_{12}$ à $C_{16}$, les alcoylaryles de $C_{10}$ à $C_{14}$ et les aryles simples sont des $C_{10}$ à $C_{16}$ Sinon la partie lipophile peut varier largement surtout lorsque le nombre de motifs éthylène glycol est au dessus de 10, elle peut ainsi constitué un radical hydrocarboné d'au moins 1, avantageusement d'au moins 3 et comportant au plus 25 avantageusement au plus 20 atomes de carbone.

Ledit composé répond à la formule I suivante.

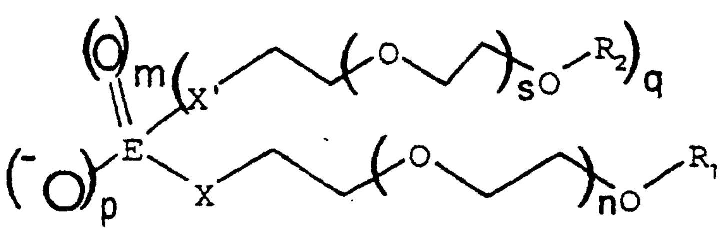

Où q représente zéro ou 1 ;

où p représente un entier entre 1 et 2 (intervalles fermés c'est à dire comprenant les bornes) ;

où m représente zéro ou un entier entre 1 et 2 (intervalles fermés c'est à dire comprenant les bornes) ;

où X et X', semblables ou différents, représente un bras comportant au plus deux chaînons carbonés ;

où s est zéro ou un entier choisi entre 1 et 30 avantageusement entre 5 et 25, de préférence entre 9 et 20 (intervalles fermés c'est à dire comprenant les bornes) ;

où n est zéro ou un entier choisi entre 1 et 30 avantageusement entre 5 et 25, de préférence entre 9 et 20 (intervalles fermés c'est à dire comprenant les bornes) ;

où E est un élément choisi parmi le carbone et les éléments métalloïdes de rang atomique au moins égal à celui du phosphore et appartenant à la colonne VB ou aux chalcogènes de rang atomique au moins égal à celui du soufre ;

où $R_1$ et $R_2$, semblables ou différents, représentent un radical hydrocarboné, avantageusement choisi parmi les aryles et les alcoyles éventuellement substitués.

Quoique cela ne fasse pas partie des composés préférés, Il convient de noter que s et/ou n peuvent être égaux à zéro, avec la condition que E est phosphore et que lorsque s et n sont égaux à zéro, respectivement R1 et/ou R2 sont alcoyles de $C_8$ à $C_{12}$, avantageusement ramifié, ou un aralcoyle de $C_{12}$ à $C_{16}$ ou un alcoyle aryle de $C_{10}$ à $C_{14}$.

L'un des radicaux divalents X et X' peut aussi être un radical de type ($[EO_m\ (O^-)_p]$) de manière à former des pyroacides comme les diesters symétrique ou non de l'acide pyrophosphorique .

Le nombre total de carbone des composés anioniques visés par la présente invention est avantageusement d'au plus environ 100, de préférence au plus environ 50.

Les radicaux divalent X et éventuellement X' sont avantageusement choisis parmi les radicaux divalents constitués par (la partie gauche de la formule étant lié au premier E) :

⇨ quand E est P, un des X ou X' peut être O-P(O)($O^-$)-X"- ;

⇨ quand E est P, un des X ou X' peut être -O-($R_{10}$-O)P(O)-X"- ; ($R_{10}$ étant défini ci après)(X" représentant un oxygène ou une simple liaison) ;

→ une liaison directe entre E et le premier éthylène dudit fragment de chaîne polyéthylène glycol ;

→ les méthylènes éventuellement substitués et dans ce cas avantageusement fonctionnalisés en parti ;

→ les bras de structure -Y- et de structure -D-Y-, -Y-D-, -Y-D-Y'

où Y et Y' représentent un chalcogène (avantageusement choisi parmi les plus légers à savoir le soufre et surtout l'oxygène), les éléments métalloïdes des rangs atomique au plus égal à celui du phosphore et appartenant à la colonne VB sous la forme de dérivés d'amines ou de phosphines tertiaires (le radical assurant le caractère tertiaire étant avantageusement d'au plus 4 carbones, de préférence d'au plus 2 carbones) ;

où D représente un alcoylène éventuellement substitué, y compris fonctionnalisé, D étant avantageusement éthylène ou méthylène, de préférence éthylène dans les structures -D-Y- et surtout -Y-D-Y', et méthylène dans les structures -Y-D-,

Ainsi E représente un atome choisi parmi les atomes de carbone (avantageusement dans ce cas m =1 et p = 1, le prototype de ce type de composé est un acide alcool [Par exemple acide lactique ou glycolique] polyéthoxylé) les atomes donnant les pnictures (éléments de la colonne VB)(avantageusement dans ce cas m = 1 ou 0 et p = 1 ou 2), les atomes de chalcogène de rang supérieur à l'oxygène (avantageusement dans ce cas m =1 ou 2 et p = 1 et q =0).

Ainsi dans le cas où E est chalcogène la formule I se simplifie avantageusement en :

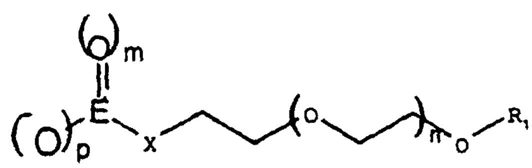

Avantageusement E représente le carbone et surtout le phosphore ou le soufre, de préférence le phosphore : dans ce dernier cas la formule (I) devient la formule (II) :

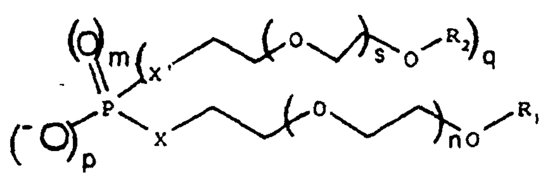

Avec lorsque q est zéro :

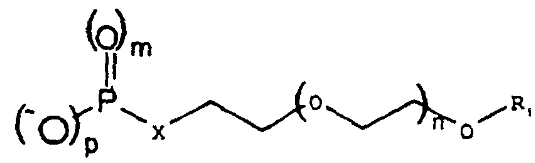

- Où p représente zéro ou un entier entre 1 et 2 (intervalles fermés c'est à dire comprenant les bornes) ;
- où m représente zéro ou un entier entre 1 et 2 (intervalles fermés c'est à dire comprenant les bornes) ;
- où la somme p + m + q est au plus égale à trois ;
- où la somme 1 + p + 2m + q est égale à trois ou à cinq ;
- où X et X', semblables ou différents, représentent un bras comportant au plus deux chaînons carbonés ;
- où n et s, semblables ou différents, représentent un entier choisi entre 5 et 30 avantageusement entre 5 et 25, de préférence entre 9 et 20 (intervalles fermés c'est à dire comprenant les bornes) ; où $R_1$ et $R_2$, semblables ou différents, représentent un radical hydrocarboné, avantageusement choisi parmi les aryles et les alcoyles éventuellement substitués notamment par atome d'halogène notamment fluor.

La classification périodique des éléments utilisée dans la présente demande est celle du supplément au Bulletin de la Société Chimique de France, janvier 1966, n° 1.
La fonctionnalisation éventuelle des alcoylènes et notamment méthylènes (X et X') est faite par des fonctions hydrophiles (amines tertiaires et autres fonctions anioniques y compris celles qui sont décrites ci-dessus [$EO_m$ $(O^-)_p$]).
Le contre-cation est avantageusement monovalents et est choisi parmi les cations minéraux et les cations organiques avantageusement non nucléophile et par voie de conséquence de nature quaternaire ou tertiaire (notamment oniums de colonne V tel que phosphonium, ammoniums, voire de colonne VI tel que sulfonium,.....) et leurs mélanges, le plus souvent ammoniums, en général issus d'une amine, avantageusement tertiaire. Avantageusement, on évite que 'le cation organique présente un hydrogène réactif avec la fonction isocyanate. D'où la préférence vis-à-vis des amines tertiaires.
Les cations minéraux peuvent être séquestrés par des agents de transfert de phases comme les éthers couronnes
Le pKa des cations (organique [ammonium.....] ou minéraux) est avantageusement compris entre 8 et 12.

- Les cations et notamment les amines correspondant aux ammoniums ne présentent avantageusement pas de propriété tensioactive mais Il est souhaitable qu'elles présentent une bonne solubilité en tout cas suffisante pour assurer celle desdits composés comportant une fonction anionique et avantageusement un fragment de chaîne polyéthylène glycol, en phase aqueuse et ce à la concentration d'emploi. Les amines tertiaires présentant au plus 12 atomes, avantageusement au plus 10 atomes, de préférence au plus 8 atomes de carbone par fonction "onium" (rappelons qu'il est préféré qu'il n'y en ait qu'une par molécule) sont préférées. Les amines peuvent comporter d'autre fonction et notamment les fonctions correspondant aux fonctions des acides aminés et des fonctions éther cyclique comme la N-méthylmorpholine, ou non. Ces autres fonctions sont avantageusement sous une forme qui ne réagisse pas avec les fonctions isocyanates et ne altèrent significativement pas la solubilité en phase aqueuse.

Il est très souhaitable que les composés anioniques selon la présente invention soient sous une forme neutralisée telle que le pH qu'elle induit lors d'une dissolution ou d'une mise en contact dans l'eau soit compris au moins égal à 3, avantageusement à 4, de préférence à 5 et au plus égal à 12, avantageusement à 11, de préférence à 10.
Lorsque E est phosphore il est souhaitable d'utiliser des mélange de monoester et de diester dans un rapport molaire compris entre 1/10 et 10, avantageusement entre 1/4 et 4. De tel mélange peuvent en outre comporter de 1 % jusqu'à environ 20 % (il est toutefois préférable que cela ne dépasse pas environ 10 %) en masse d'acide phosphorique (qui sera avantageusement salifié au moins en partie de manière à être dans les zones de pH préconisée). et de 0 à 5 % d'esters de l'acide pyrophosphorique.
Le rapport massique entre les composés tensioactifs (y compris ledit composé comportant une fonction anionique et avantageusement un fragment de chaîne polyéthylène glycol) et les isocyanates est très préférablement compris entre 4 et environ 10 %. les zones préconisées seront explicitée ultérieurement.
La composition peut comporter en outre un catalyseur avantageusement latent (libérable par action des agents extérieurs, par exemple rayonnement visible ou U.V., oxygène).
La composition d'isocyanate selon l'invention peut comporter après mise en dispersion ou émulsion dans une phase aqueuse, une teneur en eau de 10 à 70%. L'émulsion est une émulsion huile dans eau.
Toutefois, au cours de l'étude qui a mené à la présente invention, en particulier dans le cas des isocyanates

aliphatiques (c'est-à-dire reliés au squelette hydrocarboné (c'est-à-dire contenant à la fois de l'hydrogène et du carbone) par l'intermédiaire d'un carbone saturé ($sp^3$) Il a été montré qu'il avait un risque d'emballement de diverses réactions lorsque certaines proportions d'eau était atteinte. Aussi est-il avisé d'éviter des compositions où le rapport massique entre d'une part la quantité d'eau dans la phase aqueuse et d'autre part la somme de l'isocyanate et du tensioactif selon l'invention, est compris entre $10^{-2}$ et un demi. Si l'on désire plus de sûreté on évitera les rapports compris entre $10^{-3}$ et 1.

Les émulsions obtenues présentent pour la partie isocyanate des $d_{50}$ au moins égal à 0,1 micromètre, plus souvent à 0,5 micromètre et elles présentent un $d_{50}$, préférentiellement un $d_{80}$, avantageusement ≤ (au plus égal à) 5 micromètres, de préférence à 3 micromètres.

La phase aqueuse de l'émulsion, sert en général de vecteur des co-réactifs polycondensables avec les fonctions isocyanates et comporte alors des composés présentant des fonctions (avantageusement au plus 4, de préférence au plus 3 fonctions cf. ce qui est expliqué ci après pour les polyol qui général mutatis mutandis à toutes les fonctionnalité de ce type) à hydrogènes réactifs, en général un ou plusieurs polyols.

Ce polyol est un polymère qui contient au moins 2 groupements hydroxyles (phénol ou alcool) ayant avantageusement un taux d'hydroxyle entre 0,5 et 5, avantageusement entre 1 et 3 % (en masse). sauf dans le cas des latex qui sera rappelé ci-après, Il comporte avantageusement au plus 4, de préférence au plus 3 fonctions alcools primaires (mais le plus souvent deux car la réticulation proprement dite [provoquée par une fonctionnalité statistiquement supérieure à deux (valeur fractionnaire possible)] est en général engendrée par les polyisocyanates). Mais il peut comporter en outre des fonctions alcooliques secondaire ou tertiaire (en général au plus environ 10, avantageusement au plus 5, plus souvent au plus deux) qui, en général, ne réagissent pas ou ne réagissent qu'après les primaires, et ce dans l'ordre primaire, secondaire et tertiaire.

Les polyoses ou polyosides (amidon, cellulose, gommes (guar, caroube, xanthane,....) diverses....) surtout sous forme solide sont à éviter. Sous la forme d'agent texturant, et dans la mesure où cela ne gène pas la mise en émulsion et la stabilité de cette dernière, il peuvent toutefois être utilisés pour donner des propriétés particulières (Par exemple thyxotropie....). Le squelette du polymère peut être de nature chimique diverse notamment acrylique, polyester, alkyde, polyuréthanne, voire amide, y compris urée.

Le polyol peut comporter des groupements anioniques, notamment carboxyliques ou sulfoniques ou ne pas comporter de groupement ionique.

Dans le cadre de la présente invention, il a été montré que la présente de fonction carboxylate anionique ($-CO_2^-$) augmentait significativement la cinétique de séchage ce qui est particulièrement intéressant pour obtenir un "hors poussière" rapide, notamment lorsque l'on opère à l'extérieur. Un effet significatif peut être noté pour un rapport d'au moins d'une fonction carboxylique pour environ 20 fonctions à hydrogène réactif [fonction alcool ou phénol], avantageusement pour un rapport d'un pour environ 10, de préférence pour un rapport d'un pour environ 5 ; il est toutefois souhaitable que ce rapport soit au plus égale à une fonction pour une fonction, de préférence d'un fonction carboxylique pour deux fonctions ol. Les contre cations des carboxylate répondent avantageusement au mêmes préférence que celles exprimée pour les contre cation du composé selon la présente invention.

Le polyol peut déjà être en milieu aqueux ou hydrosoluble ou hydrodispersable.

Il peut s'agir d'une solution aqueuse (qui peut notamment être obtenue après neutralisation des groupements ioniques) ou d'une émulsion du polymère dans l'eau ou d'une dispersion de type latex.

Il semble possible de disperser un polyisocyanate standard dans un polyol hydrosoluble dans certaines conditions de formulation (notamment avec un rapport pigment sur liant de la peinture adapté). Mais l'utilisation de polyisocyanates standards avec des polyols hydrodispersés (types émulsion de résine ou latex), pose souvent des problèmes d'incompatibilité (floculation, apparition de plusieurs phases...). Un des nombreux avantages de la préparation selon l'invention est qu'elle offre une grande liberté de choix pour la formulation (forme physique du polyol, rapport pigment sur liant, facilité d'incorporation dans les milieux aqueux).

D'autre part il a été constaté au travers des valeurs d'usages des revêtements (notamment résistance chimique et dureté) que la réticulation des films était beaucoup plus importante quand le polyol utilisé était carboxylé.

En particulier on peut utiliser avantageusement les latex, notamment les nanolatex (c'est-à-dire latex dont la taille particulaire est nanométrique [plus précisément dont le $d_{50}$ est au plus égal à environ 100 nanomètres])

Ainsi, selon une des mises en oeuvre particulièrement avantageuses de la présente invention, le polyol est avantageusement un latex de taille nanométrique présentant les caractéristiques suivantes :

$d_{50}$ compris entre 15 et 60 nm, avantageusement entre 20 et 40 nm
fonction carboxylate de 0,5 à 5 % en masse
fonction ol : entre 1 et 4 % avantageusement entre 2 et 3 %
taux de solide : entre 25 et 40 %
un $d_{80}$ inférieur à 1 micromètre

En outre les latex, surtout quand leur point de transition vitreuse est inférieur à 0°C, avantageusement à -10°C, de préférence à -20°C, permettent l'obtention même avec des isocyanates aromatiques de bonne qualité de résistance aux intempéries et notamment aux variations de température.

Le rapport molaire entre les fonctions isocyanates libres et les fonctions hydroxyle est compris entre 0,5 et 2,5, avantageusement entre 0,8 et 1,6, avantageusement entre 1 et 1,4.

Les latex (non fonctionnalisés en isocyanate éventuellement masqués) décrits dans la demande de brevet d'invention français déposé le 28 avril 1995 N° 95/05123 et dans la demande réflexe de brevet Européen N°EP 0.739.961 donnent de forts bons résultats.

Ainsi avantageusement les particules de latex présentent une teneur en fonction acide (avantageusement carboxylique), accessible comprise entre 0,2 et 1,2 milliéquivalent/gramme de matière solide et qu'elles présentent une teneur en fonction alcoolique accessible comprise entre 0,3 et 1,5 milliéquivalent/gramme.

Ainsi, comme indiqué dans ce document sont préférés les latex constitués de particules porteuses de fonction (s) selon l'invention, sont hydrophobes et possèdent avantageusement une taille ($d_{90}$) généralement comprise entre 0,01 micromètre et 10 micromètres et de préférence au plus égale à 5 micromètres, voire à 2 micromètres. Elles sont calibrées, monodispers(é)es et présentes dans le latex à raison d'une quantité variant entre 0,2 à 65 % en poids du poids total du latex.

La masse moléculaire moyenne en poids ($M_w$ déterminée de préférence par chromatographie de perméation de gel, connue sous l'acronyme anglo-saxon de "GPC") des polymères constitutifs des particules de la population A (latex à fonction ol jouant le rôle de polyol) est avantageusement comprise entre $5.10^4$ et $5.10^6$, de préférence 0,8 $10^5$ et $2.10^6$..

Les fonctions alcooliques ou les fonctions acides, de préférence carboxyliques peuvent être également obtenues par hydrolyse de fonctions alcoologènes (ester, éther, halogénure...) ou de fonctions acidpgènes (ester, anhydride, chlorure d'acide, amide, nitrile...).

La répartition entre les différents types de motifs répond avantageusement aux règles suivantes :

La teneur en motif issu du monomère constitué par ledit alcool libre présentant une fonction éthylénique activée, et rapportée à la totalité des motifs de toute sorte, est avantageusement comprise entre 3 et 15 %, de préférence entre 4 et 10 % (mole, ou équivalent).

Selon un mode avantageux de la présente invention, le motif est issu d'un ester, d'un acide alpha éthylénique, avec un diol dont une des fonctions alcool reste non estérifiée. Ledit diol est avantageusement un diol oméga/oméga prime, avantageusement choisi parmi le butanediol-1,4, le propane diol-1,3 et le glycol.

Il est souhaitable que ledit acide alpha éthylénique soit un acide acrylique éventuellement substitué.

Selon un mode préféré de la présente invention, la teneur en motif issue d'un acide carboxylique libre (ou sous forme d'un de ses sels), et rapportée à la totalité des motifs de toute sorte, est comprise entre 2 et 10 % (mole).

Pour des raisons économiques, il est souvent avantageux que ledit acide libre soit un acide acrylique éventuellement monosubstitué ou un de ses sels.

Les particules issues de la présente invention peuvent être constituées de deux polymères distincts, le premier constituant le coeur et le second constituant la périphérie. Ce type de particule peut être obtenu par épipolymérisation [où une semence de latex est recouverte par polymérisation superficielle (épipolymérisation, parfois désignée par surpolymérisation)] d'un polymère distinct. Le coeur est parfois appelé germe par analogie au phénomène de cristallisation. Dans ce cas là, seul le deuxième polymère, c'est-à-dire le polymère superficiel, répond aux contraintes de concentration aux différentes fonctions selon la présente invention.

Le rapport massique entre ledit composés comportant une fonction anionique et avantageusement un fragment de chaîne polyéthylène glycol et les isocyanates à mettre en suspension (2 occurences) est le plus souvent au plus égal à 1/3 avantageusement au plus égal à environ 20%, de préférence à environ 10 %. (dans la présente description le terme "environ" est employé pour mettre en exergue le lait que, lorsque le, ou les, chiffres les plus à droite d'un nombre sont des zéros, ces zéros sont des zéros de position et non des chiffres significatifs, sauf bien entendu s'il en est précisé autrement).

Le rapport massique entre ledit composé comportant une fonction anionique et avantageusement un fragment de chaîne polyéthylène glycol et les isocyanates à mettre en suspension (2 occurences) est avantageusement supérieur à 1 %, de préférence à 2 %.

Il est également souhaitable que la quantité dudit ou desdits composés comportant une fonction anionique et avantageusement un fragment de chaîne polyéthylène glycol corresponde à une valeur comprise entre $10^{-2}$ et 1, avantageusement entre 5.10-2 et 0,5 atome de E par litre

Ainsi le rapport massique entre ledit composé comportant une fonction anionique et avantageusement un fragment de chaîne polyéthylène glycol et les isocyanates à mettre en suspension est avantageusement au moins égal à 2 %, de préférence à 4 %, et au plus égal à environ 20 %, de préférence à environ 10 %, ainsi ce rapport massique est avantageusement compris entre et environ 20 %, de préférence entre 4 et environ 10 %.

Selon une mise en oeuvre particulièrement avantageuse de la présente invention, après mise en dispersion

ou émulsion, la somme des constituants du liant (c'est-à-dire des teneurs en masse du, ou des, isocyanates, des émulsifiants et des polyols), dans l'eau varie de 30 à 70 % par rapport à la totalité de la composition.

Les isocyanates visés par l'invention comporte notamment les composés détaillés ci après.

Ces composés peuvent avantageusement contenir les structures courantes dans ce domaine par exemple les prépolymères issus de la condensation de polyol (Par exemple triméthylol propane) en général triol (avantageusement primaire voire plus loin sur la définition des polyols) et surtout les plus courantes, à savoir celles de type isocyanurate, encore appelée trimère, des structures urétidine dione, encore appelée dimère, des structures biuret ou allophanate ou une combinaison de ce type de structures sur une seule molécule ou en mélange.

Si l'on désire abaisser fortement la teneur en solvant de la composition, notamment lorsqu'elle sous forme d'émulsion, il est préférable d'utiliser des mélanges de ce type naturellement (c'est-à-dire sans adjonction de solvant) à basse viscosité. Les composés présentant cette propriété sont surtout les dérivés (type isocyanurate, encore appelée trimère, des structures urétidine dione, encore appelée dimère, des structures biuret ou allophanate ou une combinaison de ce type de structures sur une seule molécule ou en mélange) en partie et/ou en totalité des isocyanates aliphatiques dont les fonctions isocyanate sont reliées au squelette par l'intermédiaire de fragments éthylène (Par exemple des polyméthylènediisocyanates, notamment l'hexaméthylènediisocyanate et ceux des arylènedialcoylènediisocyanates dont la fonction isocyanate est distante des noyaux aromatique d'au moins deux carbones tels que ($OCN-[CH_2]_t-\Phi-[CH_2]_u-NCO$) avec t et u supérieur à 1). Ces composés ou mélanges ont avantageusement un viscosité au plus égale à environ 3000 centipoises (ou millipascal.seconde), de préférence à environ 1500 centipoises (ou millipascal.seconde).

Lorsque ces valeurs ne sont pas atteintes Il est alors souvent utile d'amener le mélange à ces valeurs de viscosité par l'adjonction d'une quantité minimale de solvant(s) approprié(s).Ainsi que déjà mentionné ci-dessus, les isocyanates concernés peuvent être des mono-, di- voire poly-isocyanates. Avantageusement, ces dérivés peuvent contenir des structures de type isocyanurate, encore appelée trimère, des structures urétidine dione, encore appelée dimère, des structures biuret ou allophanate ou une combinaison de ce type de structures sur une seule molécule ou en mélange.

Les isocyanates monomères peuvent être :

⇨ aliphatiques, y compris cycloaliphatiques et arylaliphatiques, tels que :

- · comme aliphatique simples, les polyméthylènediisocyanates et notamment l'hexaméthylène diisocyanate ;
- · comme aliphatique partiellement "néopentylique" partiellement cyclique (cycloaliphatique) l'isophorone diisocyanate (IPDI) ;
- · comme aliphatique cyclique (cycloaliphatique) diisocyanate ceux dérivés du norbornane ;
- · les arylènedialcoylènediisocyanates (tel que $OCN-CH_2-\Phi-CH_2-NCO$ dont une partie ne présente pas de différence essentielle d'avec les aliphatiques à savoir ceux dont la fonction isocyanate est distante des noyaux aromatique d'au moins deux carbones tels que ($OCN-[CH_2]_t-\Phi-[CH_2]_u-NCO$) avec t et u supérieur à 1 ;

⇨ ou encore aromatiques tels que le toluylène diisocyanate.

Les polyisocyanates préférés visés par la technique de l'invention sont ceux dans lesquels au moins une, avantageusement deux, de préférence trois des conditions ci après sont remplies :

· Au moins une, avantageusement au moins deux, des fonctions NCO sont reliées à un squelette hydrocarboné, par l'intermédiaire d'un carbone saturé ($sp^3$), de préférence avec au moins une, plus préférentiellement au moins deux des sous conditions ci- après :.

- · Au moins un, avantageusement deux, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes) ;

· au moins un, avantageusement deux, desdits carbones saturés ($sp^3$) sont eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite "néopentylique" ;

- · Tous les carbones par l'intermédiaire desquels les fonctions isocyanates sont reliées au squelette hydrocarboné, sont des carbones saturés ($sp^3$), lesquels sont avantageusement en partie, de préférence en totalité, porteurs d'un hydrogène, de préférence de deux hydrogènes ; en outre il est avantageux que lesdits carbones

saturés ($sp^3$) soient au moins en partie (avantageusement un tiers, de préférence deux tiers), préférablement en totalité, eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite "néopentylique" ;

· Sont en particulier bien adaptés ceux qui présentent au moins en partie un squelette isocyanurique ou biuret (que ce squelette soit issu d'un seul ou de plusieurs monomères, voir ci-dessous) et plus précisément des structures de type isocyanurate, encore appelée trimère, des structures urétidinedione, encore appelée dimère, des structures biuret ou allophanate ou une combinaison de ce type de structures sur une seule molécule ou en mélange.

Lorsque les polyisocyanates sont relativement lourds c'est à dire qu'ils comportent au moins 4 fonctions isocyanates, les deux premières conditions deviennent :

· Au moins un tiers, avantageusement deux tiers, des fonctions NCO sont reliées à un squelette hydrocarboné, par l'intermédiaire d'un carbone saturé ($sp^3$) ;

· Au moins un tiers, avantageusement deux tiers, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes.; En outre il est avantageux que lesdits carbones saturés ($sp^3$) soient au moins en partie (avantageusement un tiers, de préférence deux tiers), préférablement en totalité, eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite "néopentylique" ;

Les isocyanates notamment aliphatiques réagissent avec certains des composés anioniques visés par l'invention il réagissent avec l'hydroxyle des fonctions acides non ou mal neutralisées. ces composé sont également visé par la présente invention.

En particulier dans le cas des phosphates (m = 1) ils réagissent pour donner des composés du type :

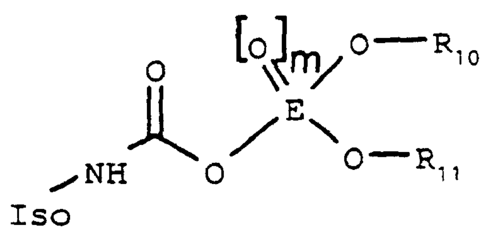

mais quand E appartient à la colonne du phosphore et que m (qui est le même que dans la formule I) est égale à zéro le composé s'isomérise (ou se fait directement) pour donner :

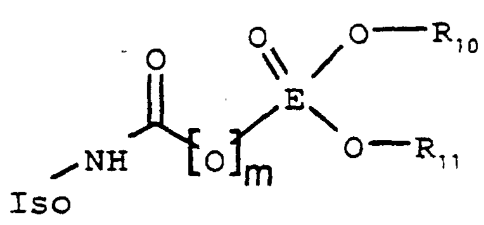

⇨ Où E est un élément de la colonne VA la classification périodique des éléments [(supplément au Bulletin de la Société Chimique de France Janvier 1966 N°1) avantageusement du phosphore]. Et donc notamment du type :

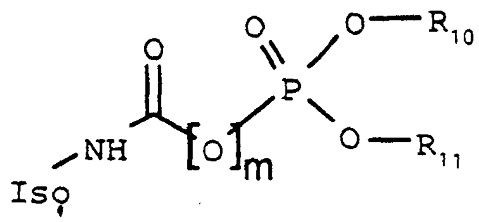

➪ Où Iso est le reste (d'un poly)isocyanate (après élimination d'une fonction isocyanate)
➪ où $R_{10}$ est un reste hydrocarboné (c'est-à-dire contenant des atomes d'hydrogène et de carbone) dont le point d'attache [c'est-à-dire l'atome porteur de la liaison ouverte] est un carbone
➪ où $R_{11}$ est choisi parmi :

→ une charge négative ;
→ un groupe de formule II :

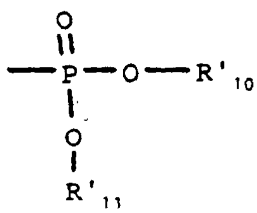

dans laquelle $R'_{10}$ est choisi parmi les restes hydrocarbonés (semblables ou différents de $R_{10}$) et une charge négative dont le point d'attache [c'est-à-dire l'atome porteur de la liaison ouverte] est un carbone
dans laquelle le(s) $R'_{11}$ est (sont) choisi(s) parmi les restes hydrocarbonés dont le point d'attache [c'est-à-dire l'atome porteur de la liaison ouverte] est un carbone (semblables ou différents de $R_{10}$ et de $R'_{11}$) et une charge négative.
Il est souhaitable qu'au moins un des substituants organiques ($R_{10}$ ; $R'_{11}$ ; $R'_{10}$) comporte un fragment de chaîne polyéthylène glycol avantageusement d'au moins 5, de préférence d'au moins 7 unités éthylènyloxyles. En d'autres termes il est souhaitable qu'au moins un des substituants organiques, réponde à la de même formule que les substituants de E dans la formule générale I. Plus spécifiquement au moins un des substituants organiques ($R_{10}$ ; $R'_{11}$ ; $R'_{10}$) répond à la formule

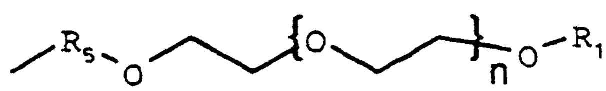

Où $R_5$ représente un bras comportant au plus deux chaînons carbonés (avec les mêmes valeurs préférées que X' et X)
où n est un entier choisi entre 0 et 30 avantageusement entre 5 et 25, de préférence entre 9 et 20 (intervalles fermés c'est à dire comprenant les bornes) ;
où $R_1$ représente un radical hydrocarboné, avantageusement choisi parmi les aryles et les alcoyles éventuellement substitués.

Ainsi selon une variante avantageuse de la présente invention, les compositions selon la présente invention présente des composés issus de la réaction exposées ci-dessus dans une proportion globale, par rapport à un volume d'un litre d'isocyanate, de 0,01 à 1, avantageusement de 0,05 à 0,5, de préférence de 0,05 à 0,3 équivalent de fonction :

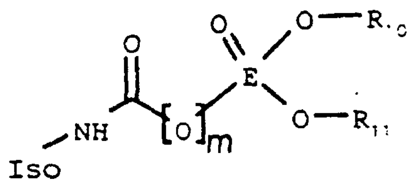

Il est avantageux que le radical Iso procure majoritairement ou en totalité un lien aliphatique avec les même préférence que celles exposées ci dessus à propos des isocyanates.

Font donc également partie de l'invention les composés de formule :

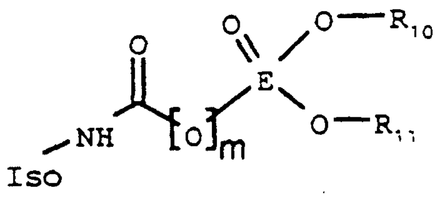

Dans laquelle R10 et R11 peuvent prendre les valeurs ci dessus mais aussi quand m est 1, être une charge négative en raison du fait que dans certain lot il peut y avoir des quantités significatives d'acide phosphorique résiduel.

Bien sur alors $R_{10}$ peut aussi valoir :

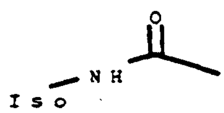

Le radical Iso pouvant ou non être alors le même que celui de l'avant dernière formule.

dans laquelle Iso représente le reste d'un polyisocyanate, avantageusement d'un produit de réaction d'un monomère diisocyanate pour former du biuret ou des isocyanurates (trimère), ou avec un di- ou poly-ol avantageusement un triol ou un tetra-ol.

Il est avantageux que le radical Iso procure majoritairement ou en totalité un lien aliphatique avec les mêmes préférences que celles exposées ci dessus à propos des isocyanates.

Avantageusement Iso porte, outre la fonction figurée sur la formule au moins une, de préférence au moins deux fonctions isocyanates, dont, de préférence, au moins une n'est pas masquée, et dont, plus préférentiellement, au moins deux ne sont pas masquées.

Un autre but de la présente invention est de fournir un procédé du type précédent qui permette de réaliser la mise en émulsion de la composition visée ci dessus lorsqu'elle ne contient pas d'eau.

Ce but et d'autres qui apparaîtront par la suite sont atteints au moyen d'un procédé de mise en émulsion qui comporte au moins l'étape suivante :

· ajout, avantageusement sous agitation très modérée, des ou de l'isocyanate dans le mélange polyol + eau.

L'agent tensioactif peut être soit dans la phase aqueuse, soit de préférence dans la phase isocyanate. Dans le premier cas les réactions entre isocyanate et ledit composé comportant une fonction anionique et avantageusement un fragment de chaîne polyéthylène glycol sont beaucoup moins importantes.

Cette agitation est de préférence manuelle ou mécanique.

Cette mise en émulsion est avantageusement menée à une température inférieure à 50°C, de préférence à l'ambiante.

Il est souhaitable de réaliser, si nécessaire, un ajustement du pH (pour atteindre une valeur avantageusement au moins égale à trois, de préférence à 4 et avantageusement au plus égal à 11 de préférence à 10 et donc avantageusement comprise entre 3 et 11, de préférence entre 4 et 10) lors de la mise en émulsion. Cet ajustement permet d'atteindre une zone avantageuse où la première (ou unique) acidité de chaque agent tensioactif selon la présente invention est neutralisée.

Selon une variante avantageuse de la présente invention, les pigments (et notamment le bioxyde de titane)

sont dispersés dans le ou les polyols avant l'addition de l'isocyanate.

Un autre but de la présente invention est de fournir un procédé d'application de la composition à base d'isocyanate pour former un revêtement.

Ces buts et d'autres qui apparaîtront par la suite sont atteints au moyen d'un procédé comportant l'application d'une couche de préparation (c'est-à-dire de composition selon l'invention comportant la phase aqueuse et les constituants de la couche) dont l'épaisseur avant séchage est comprise entre 10 et 400 micromètres avantageusement entre 50 et 200 correspondant après séchage à une épaisseur comprise entre 5 et 150 micromètres, avantageusement entre 20 et 80 micromètres.

Selon une mise en oeuvre avantageuse, ce procédé comporte un séchage de 20°C à 60°C pendant une durée pouvant aller de 1/4 à 24 heures.

Avantageusement ce séchage a lieu en présence d'un solvant pour aider à l'élimination de l'eau.

Selon une mise en oeuvre particulièrement avantageuse de la présente invention, l'application est menée par pulvérisation.

La préparation des surfaces est bien connue de l'homme de métier (Par exemple phosphatations pour les composé ferreux acier ou chromatation pour les surface a base d'alumine). (on peut par exemple se référer au ouvrage suivants : "organic coating technology " volume II de H.F. PAYNE et "Paint Handbook" Edité par G.E.WEISMANTEL)

Selon la présente invention, il est ainsi possible d'obtenir des revêtements (notamment peintures ou vernis) présentant les caractéristiques techniques (ces valeur dépendent surtout des polyols utilisés) suivantes :

| mise en oeuvre et caractéristiques du revêtement<br>épaisseur sèche Iso2178 : 45 µm<br>support et traitement de celui-ci :acier traité par phosphatation : plaques R46I<br>du fournisseur Q Pannel | | |
|---|---|---|
| | propriétés minimales obtenues | usuelle |
| test din 67530 (ces valeurs n'ont d'intérêt que lorque l'on désire une peinture brillante mais non lorsque l'on veut de la peinture mat ou satinée)<br>Brillance 20°<br>60° | <br><br>0,5<br>0,5 | <br><br>80<br>90 |
| dureté König iso 1522 | 10s | 150s |
| Adhérence test din 53151 | GT-1 | GT-5 |
| Résistance au chocs test<br>N°iso 6272<br>direct<br>inverse | 10 cm<br><br><br>5 cm | >100 cm<br><br><br>>100 cm |
| Résistance à la méthyl-éthylcétone (butanone) (Double passage) | 20 | >200 |
| Tenue en extérieur QUV<br>Din 53384 | 50 h | 800 h |

Les exemples non limitatifs suivants illustrent l'invention.

Le Rhodafac® RE610 est un mélange de mono- et de diesters phosphorique selon la formule II, la formule moyenne de son radical hydrocarboné est un nonylphénol polyéthoxylé (~dix fois). le rapport molaire entre mono ester et diester est de environ 1 (arrondi mathématique). De même le Rhodafac® PA17 présente comme produit selon la présente invention un mélange de mono- et de diesters phosphorique selon la formule II, la formule moyenne de son radical hydrocarboné est un nonylphénol polyéthoxylé (-cinq-six fois).

**Exemple 1** - préparation du mélange 1

on mélange 165g de tolonate® HDT (oligomère isocyanurate à base de trimère) avec 24g d'acétate de butyle et 13g de Rhodafac® RE610 (mélange de mono- et de diesters phosphorique selon la formule II)et 3g de triéthylamine. Ce mélange est agité à l'aide d'une pale cadre ou défloculeuse pendant 5 minutes à 100 tours/minute. Ce mélange a une viscosité de 0,84 Pa.s. A 20°C et une coloration inférieure à 100 APHA.

**Exemple 2** - préparation du mélange 2

le mélange a la même composition que le mélange 1 mais le tolonate® HDT est remplacé par le tolonate® HDT-LV.

La viscosité de ce mélange est de 0,476Pa.s. A 20°C et une coloration inférieure à 100 APHA.

**Exemple 3** - préparation du mélange 3

le mélange est fait avec 92g de tolonate® HDT et 10g de Rhodafac RE610 et 2,3g de triéthylamine. La viscosité de ce mélange est de 5,2 Pa.s. A 20°C et la coloration inférieure à 100 APHA.

**Exemple 4 -**

30g du mélange 1 est ajouté à 20g d'eau. Le mélange est agité à l'aide d'une pale cadre à 250 tours/min pendant 5 minutes. On obtient ainsi une émulsion dont la granulométrie moyenne est 1,2 µm. La taille des gouttes d'émulsion reste stable pendant 30 heures à un pH situé entre 3 et 9.

**Exemple 5**

30g du mélange 3 est ajouté à 20g d'eau. L'émulsion est préparée dans les mêmes conditions que dans l'exemple 4. La taille moyenne des gouttes est de 1,1 µm .

**Exemple 6 comparatif**

30g d'un mélange HDT-LV / acétate de butyle (même rapport pondéral que dans l'exemple 2) est ajouté à 20 g d'eau. L'émulsion est préparée dans les mêmes conditions que dans l'exemple 4. On obtient ainsi une émulsion grossière dont la taille moyenne est supérieure à 5µm et donc difficile à caractériser.

**Exemple 7 (comparatif)**

On mélange 165g de tolonate® HDT avec 13 g de Rhodocal® AT60 (dodecylbenzène sulfonate de triéthylamine). Ce mélange est agité à l'aide d'une pale cadre à 100 tours/min. Pendant 5 minutes. 10g de ce mélange est ajouté à 90 g d'eau sous agitation à l'aide d'une pale cadre à 400 tours/min. Pendant 10 minutes. Ensuite le produit est analysé, il est constitué de deux phases, l'une riche en huile et l'autre riche en eau mais il n'y a pas formation d'une émulsion homogène.

**Exemple 8**

On mélange 83 g de tolonate® HDB (biuret) avec 6g de Rhodafac® PA17 et 1,2g de triéthylamine et 10g d'acétate de butyle. Ce mélange est homogénéisé à l'aide d'une pale cadre à 100 tours/min. Pendant 5 minutes.

10 g de ce mélange est ajouté à 90 g d'eau sous agitation à l'aide d'une pale cadre à 200 tours/min. Pendant 5 minutes. L'émulsion obtenue présente une granulométrie centrée autour de 3,5µm.

**Exemple 9**

on mélange 83g de tolonate® HDT avec 6g de Rhodafac® PA17 et 1,2g de triéthylamine et 10g d'acétate de butyle. Ce mélange est homogénéisé à l'aide d'une pale cadre à 100 tours/min. Pendant 5 minutes.

10 g de ce mélange est ajouté à 90 g d'eau sous agitation à l'aide d'une pale cadre à 200 tours/min. Pendant 5 minutes. L'émulsion obtenue présente une granulométrie centrée autour de 0,98µm.

**Exemple 10**- Préparation d'un vernis à partir du mélange 1 et d'un polyol commercialisé par la société SC JOHNSON POLYMER sous le nom de JONCRYL® 540.

Ce polyol est une émulsion de copolymères acryliques d'extrait sec 42% et d'indice d'hydroxyle 42 (sur matière sèche).

On prépare un vernis par incorporation de 7,3 g du mélange 1 dans 92,7 g de JONCRYL® 540. L'incorporation se fait sous agitation manuelle avec une spatule pendant 10 minutes. Les proportions entre le mélange 1 et le polyol sont telles que le rapport molaire NCO/OH soit égal à 1/1.

Des films de vernis ont été appliqués avec une tige filetée (de façon à avoir une épaisseur sèche de 40µm) 1/2 heure et 4 heures après l'incorporation de l'isocyanate dans le polyol.
Les performances obtenues à 1/2 heure et 4 heures de vieillissement du mélange, après 48 heures de séchage à 23°C et 55% d'humidité relative, sont équivalentes et d'un bon niveau : brillance de 89 sous un angle de 20°, résistance à la Méthyl Ethyl Cétone supérieure à 100 aller-retour avec un coton imbibé, dureté pendulaire Persoz égale à 140 s.

**Exemple 11** - Préparation d'une peinture blanche brillante à partir du mélange 2 et d'un polyol

Une peinture sera constituée de 2 composants : un élément A contenant notamment et le plus souvent majoritairement, le pigment et le polyol, et un élément B constitué uniquement du mélange 2.
Le polyol utilisé dans le présent exemple est celui commercialisé par la société JAGER sous le nom de JAGOTEX® F 313.
Ce polyol est une solution aqueuse d'une résine de copolymères acryliques contenant des groupes acides et hydroxyles. Il est neutralisé avec de la Di Méthyl Ethyl Amine à un pH de 8,5, il a un extrait sec de 45%, un indice d'acide de 60 et un indice d'hydroxyle de 132.
100g d'élément A est obtenus par mélange sous agitation poussée (20 minutes à 1000 tours/mn avec une paie défloculeuse) de 49,7 g de JAGOTEX® F 313, de 0,8 g de DISPERBYK® 181 (agent mouillant commercialisé par la société BYK), de 0,3 g de BYK® 022 (agent antimousse commercialisé par la société BYK), de 23,7 g de TITAFRANCE® RL 60 (pigment de bioxyde de titane commercialisé par la société RHONE-POULENC) et de 25,5 g d'eau déminéralisée.
Une peinture est préparée par incorporation sous agitation réduite (environ 300 tours/mn avec pale défloculeuse) de 3,9 g d'élément B (mélange 2) dans le composant A. Ces proportions sont telles que le rapport Pigment/ Liant de la peinture soit égal à 0,75/1 et que le rapport NCO/OH dans la peinture soit égal à 1/1.
Des films de peinture ont été appliqués sur des plaques d'acier avec une tige filetée (de façon à avoir une épaisseur sèche de 30µm) 1/2 heure après l'incorporation de l'isocyanate dans le polyol. La durée d'utilisation de la peinture (mélange A + B) est de 3 heures.
Les performances des films, après 48 heures de séchage à 23°C et 55% d'humidité relative, sont d'un bon niveau : brillance de 71 sous un angle de 20° et 84 sous un angle de 60°, résistance à la Méthyl Ethyl Cétone supérieure à 100 aller-retour avec un coton imbibé, dureté pendulaire Persoz égale à 100 s, temps de séchage "sec au toucher" égal à environ 3 heures à 23°C et 55 % d'humidité relative.

**Exemple 12** - Préparation d'une peinture blanche à partir du mélange de l'exemple 6 et du polyol JAGOTEX® F 313 commercialisé par la société JAGER (cf. Caractéristiques données dans l'exemple 12).

La peinture sera obtenue par mélange d'un élément A contenant le pigment et le polyol, et d'un élément B constitué uniquement du mélange de l'exemple 6.
L'élément A est préparé selon le procédé et la formule de l'exemple 11.
La peinture est préparée par incorporation sous agitation réduite (environ 300 tours/mn avec pale défloculeuse) de 3,6 g d'élément B (mélange de l'exemple 6) dans le composant A. Ces proportions sont telles que le rapport Pigment/Liant de la peinture soit égal à 0,75/1 et que le rapport NCO/OH dans la peinture soit égal à 1/1 (conditions identiques à celles de l'exemple 11).
Des films de peinture ont été appliqués dans les mêmes conditions que celles de l'exemple 11
Les performances des films, après 48 heures de séchage à 23°C et 55% d'humidité relative, sont médiocres : brillance de 8 sous un angle de 20° et 27 sous un angle de 60° et résistance à la Méthyl Ethyl Cétone inférieure à 10 aller-retour avec un coton imbibé.
La comparaison de cet exemple et de l'exemple 11 montre la potentialisation apportée par la combinaison avec tensioactif Rhodafac RE 610 notamment neutralisé avec la triéthylamine.

**Exemple 13** - utilisation de Nanolatex:

Préparation d'un vernis à partir du mélange 1 et d'un nanolatex_(c'est-à-dire latex dont la taille particulaire est Nanométrique [plus précisément dont le $d_{50}$ est au plus égal à environ 100 nanomètres]) de monomères acryliques.
Le nanolatex utilisé est un produit expérimental réalisé selon le procédé décrit dans la demande de brevet d'invention français déposé le 28 avril 1995 N° 95/05123 et dans la demande réflexe de brevet Européen N°EP 0.739.961 de caractéristiques suivantes :

$d_{50}$ : environ 35 nm

fonction carboxylate : 2,6 % en poids sur le polymère sec
fonction of : 2,6 % en poids sur le polymère sec
Masse moléculaire supérieure à 100 000
Extrait sec en poids : 30 %
pH environ 8
Température Minimum de Formation de Film environ 20°C
Température de transition vitreuse environ 40°C

Le vernis est préparé par incorporation avec mélange manuel de 4, 6 g du mélange 1 dans 45,6 g de nanolatex. Ce ratio correspond à un rapport des fonctions NCO/OH de 1,2.
Le mélange ainsi préparé présente une durée de vie de 4 heures, ceci signifie que pendant 4 heures la viscosité et l'aspect du mélange est inchangé mais aussi que les films formés à partir de ce mélange pendant ces 4 heures ont des propriétés comme la résistance au solvant, la dureté et la brillance inchangés.
Le temps de séchage du film est remarquablement court : 20 minutes pour le temps "hors poussière" et 30 minutes pour le temps "sec au toucher" selon la norme NF T 30037. Ces mesures ont été faites sur plaques de verre pour une épaisseur sèche de film de 40 µm et un séchage à 23°C avec 55% d'humidité relative.
Le fait d'obtenir un temps de séchage court et un durée de vie du mélange relativement long constitue un avantage très recherché par les experts en peinture.
Les valeurs d'usage du revêtement sont également globalement d'un très bon niveau, on peut citer:

- la dureté Persoz du film de 50 µm sec mesurée sur plaque d'acier après séchage de 24 heures à 23°C et 55 % d'humidité relative est égale à 275 secondes.
- la brillance sous un angle de 20° du film appliqué sur plaque d'acier est égale à 90
- la résistance à la Méthyl Ethyl Cétone du film séché pendant 7 jours à 23°C et 55 % d'humidité relative est supérieure à 200 aller-retour avec un coton imbibé.

Ces excellentes performances de cette association Nanolatex-polyisocyanate selon l'invention sont certainement imputables à la physico-chimie très particulière de ces produits notamment à la très importante surface spécifique du nanolatex qui favorise une grande homogénéité de réticulation dans le polymère final.

**Exemple 14**

Le mélange préparé dans l'exemple 13 est appliqué à la brosse avec un dépôt de 200 g/m2 sur un support de type béton.
Après séchage de 1 h à température ambiante, le revêtement offre une hydrofugation du support qui se traduit par un effet perlant, et une moindre absorption d'eau. De plus le revêtement présente une bonne résistance à l'abrasion.

**Exemple 15** -

Préparation d'un revêtement à partir du mélange 1 et d'un nanolatex de monomères acryliques.
Le nanolatex est un produit expérimental selon la demande d'invention N° de caractéristique suivante :

$d_{50}$ environ 35 nm
fonction carboxylate : 1 % en poids sur le polymère sec
fonction ol : 2,6 % en poids sur le polymère sec
Masse moléculaire supérieure à 100 000
Extrait sec en poids : 30 %
pH environ 8
Température de transition vitreuse environ - 30°C

Le vernis est préparé par incorporation avec mélange manuel de 4, 6 g du mélange 1 dans 45,6 g de nanolatex. Ce ratio correspond à un rapport des fonctions NCO/OH de 1,2.
Le revêtement est appliqué sur béton à la brosse à raison de 2 couches de 300 g/m2. Sa grande souplesse, générée principalement par le faible Tg du nanolatex, lui confère une capacité à masquer les fissure pouvant apparaître lors du vieillissement du support (notamment à cause de la dilatation du béton à cause de grande variation de température).
De plus la réticulation avec le polyisocyanate selon l'invention confère au revêtement une excellente résistance aux agents chimiques et à l'eau.

## Revendications

**1.** Composition à base d'isocyanate(s) comprenant :

- un composé (poly)isocyanate non masqué ou partiellement masqué, dans lequel au moins une des fonctions NCO est reliée à un squelette hydrocarboné, par l'intermédiaire d'un carbone saturé ($sp^3$) ; et
- un tensioactif anionique de formule générale I suivante :

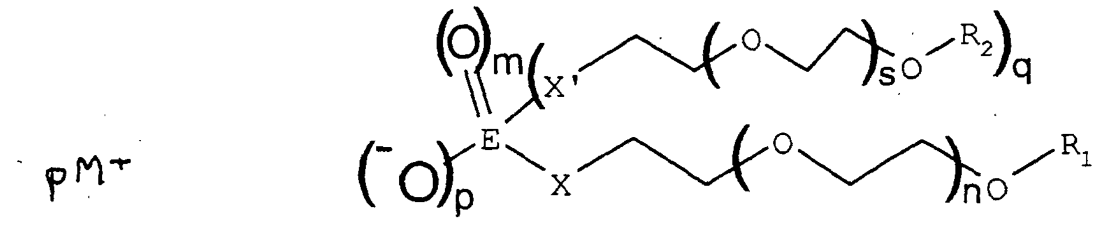

où q représente zéro ou 1 ;
où p représente un entier entre 1 et 2 (intervalles fermés c'est à dire comprenant les bornes) ;
où m représente zéro ou un entier entre 1 et 2 (intervalles fermés c'est à dire comprenant les bornes) ;
où X et X', semblables ou différents, représente un bras comportant au plus deux chaînons carbonés ;
où s est zéro ou un entier choisi entre 1 et 30 ;
où n est zéro ou un entier choisi entre 1 et 30,
avec la condition que lorsque s et/ou n sont égaux à zéro, E est phosphore et que lorsque s et n sont égaux à zéro, $R_1$ et/ou $R_2$ sont alcoyles en $C_8$-$C_{12}$ ou aralcoyle en $C_{12}$-$C_{16}$ ou alcoylaryle en $C_{10}$-$C_{14}$ ;
où E est un élément choisi parmi le carbone et les éléments métalloïdes de rang atomique au moins égal à celui du phosphore et appartenant à la colonne VB ou aux chalcogènes de rang atomique au moins égal à celui du soufre ;
où $R_1$ et $R_2$, semblables ou différents, représentent un radical hydrocarboné, éventuellement substitué ;
où $M^+$ est un contre-cation,
ladite composition étant exempte de composés hydroxylés polyfonctionnels d'un poids moléculaire moyen en nombre de 400 à 5000 ;
ou ladite composition comprenant

- un composé (poly)isocyanate non masqué ou partiellement masqué, dans lequel au moins une des fonctions NCO est reliée à un squelette hydrocarboné, par l'intermédiaire d'un carbone saturé ($sp^3$) au moins partiellement sous la forme

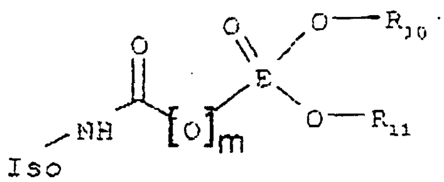

où m est le même que dans la Formule I

- ⇨ où E est un élément de la colonne VA la classification périodique des éléments ;
- ⇨ où Iso est le reste d'un (poly)isocyanate ;
- ⇨ où $R_{10}$ est une charge négative ou un reste hydrocarboné contenant des atomes d'hydrogène et de carbone lié à l'atome d'oxygène par un carbone ;
- ⇨ où $R_{11}$ est choisi parmi :
  - ⇓ une charge négative ;
  - ⇓ un groupe de formule :

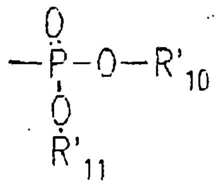

dans laquelle R'$_{10}$ est choisi parmi une charge négative et les restes hydrocarbonés semblables ou différents de $R_{10}$ et lié à l'atome d'oxygène par un carbone

dans laquelle le(s) R'$_{11}$ est (sont) choisi(s) parmi une charge négative et les restes hydrocarbonés semblables ou différents de $R_{10}$ et de R'$_{11}$ et liés par un atome d'oxygène par un atome de carbone

sous réserve qu'au moins un des substituants $R_{10}$, R'$_{10}$ et R'$_{11}$ comporte un fragment de chaîne polyéthylène glycol d'au moins 1, avantageusement d'au moins 5, de préférence d'au moins 7 unités éthylényloxyles,

et éventuellement un composé de formule I tel que défini ci-dessus.

**2.** Composition à base d'isocyanate(s) selon la revendication 1, **caractérisée en ce que** tous les atomes de carbone par l'intermédiaire desquels les fonctions isocyanates sont reliées au squelette hydrocarboné sont des atomes de carbones saturés $sp^3$.

**3.** Composition selon la revendication 1 ou 2, **caractérisée en ce que** :

- $R_1$ et $R_2$ représentent des groupes ($C_8$-$C_{12}$) alcoyles, des groupes aralcoyles en $C_{12}$-$C_{16}$, des groupes $C_{10}$-$C_{14}$ alcoylaryles et des groupes $C_{10}$-$C_{16}$ aryles, lorsque le nombre de fonctions oxydes d'éthylène dans la formule I est au plus égal à 5 ; ou
- $R_1$ et $R_2$ représentent un groupe hydrocarboné ayant au moins 1 et au plus 25 atomes de carbone, lorsque le nombre de fonctions oxydes d'éthylène est supérieur à 5.

**4.** Composition selon l'une quelconque des revendications 1 à 3, **caractérisée en ce que** l'un des X et éventuellement des X' sont choisis parmi les radicaux divalents constitués par :

- O-P(O)(O-)-X"-, quand E est P ;
- -O-($R_{10}$-O)P(O)-X"-, quand E est P, X" représentant un oxygène ou une simple liaison ;
- une liaison directe entre E et le premier éthylène dudit fragment de chaîne polyéthylène glycol ;
- un groupe méthylène éventuellement substitué ;
- un bras de structure -Y- et de structure -D-Y-, -Y-D- ;

où Y représente un chalcogène, les éléments métalloïdes des rangs atomique au plus égal à celui du phosphore et appartenant à la colonne VB sous la forme de dérivés d'amines ou de phosphines tertiaires ;

où D représente un alcoylène éventuellement substitué, y compris fonctionnalisé.

**5.** Composition selon la revendication 4, **caractérisée en ce que** l'un des X et X' est un radical de formule [$EO_m$ $(O^-)_p$], m et p étant tels que définis ci-dessus.

**6.** Composition selon l'une des revendications précédentes, **caractérisée en ce que** E est choisi parmi carbone, phosphore et soufre.

**7.** Composition selon la revendication 6, **caractérisée en ce que** E est phosphore.

**8.** Composition selon l'une quelconque des revendications précédentes, **caractérisée en ce que** le composé anionique est représenté par la formule II :

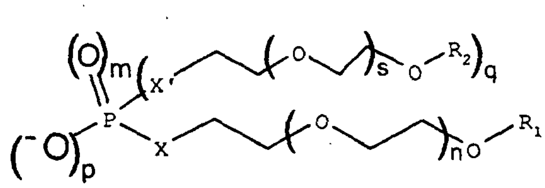

et lorsque q est zéro :

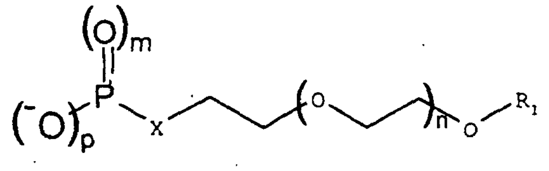

- où p représente zéro ou un entier entre 1 et 2 ;
- où m représente zéro ou un entier entre 1 et 2 ;
- où la somme p + m + q est au plus égale à trois ;
- où la somme 1 + p + 2m + q est égale à trois ou à cinq ;
- où X et X', semblables ou différents, représentent un bras comportant au plus deux chaînons carbonés ;
- où n et s, semblables ou différents, représentent un entier choisi entre 5 et 30 ;
- où $R_1$ et $R_2$, semblables ou différents, représentent un radical hydrocarboné, éventuellement substitué.

**9.** Composition selon la revendication 1, **caractérisée en ce que** lorsque E est phosphore, le composé anionique consiste en un mélange de monoester et de diester dans un rapport molaire compris entre 1/10 et 10, pouvant en outre comporter de 1 % jusqu'à environ 20 % en masse d'acide phosphorique et de 0 à 5 % d'esters d'acide pyrophosphorique.

**10.** Composition selon la revendication 1, **caractérisée en ce que** le rapport massique entre les composés tensioactifs et les isocyanates est compris entré 4 et 10 %.

**11.** Composition selon les revendications précédentes, **caractérisée par le fait que** le contre cation est une amine, avantageusement tertiaire.

**12.** Composition selon les revendications 1 à 10, **caractérisée par le fait que** la composition comporte en outre un catalyseur avantageusement latent.

**13.** Emulsion ou suspension aqueuse comprenant :

- un composé (poly)isocyanate non masqué ou partiellement masqué, dans lequel au moins une des fonctions NCO est reliée à un squelette hydrocarboné, par l'intermédiaire d'un carbone saturé ($sp^3$); et
- un agent tensioactif de formule I suivante :

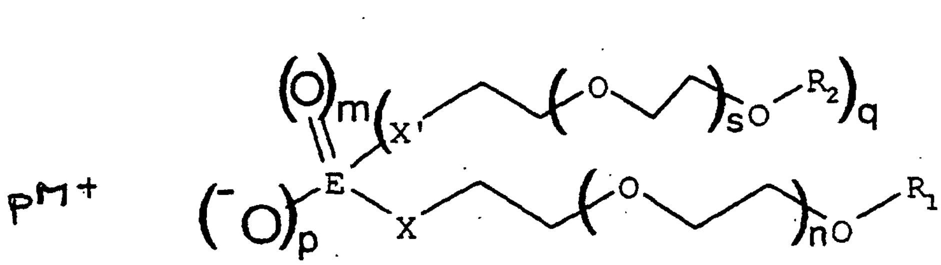

où q représente zéro ou 1 ;
où p représente un entier entre 1 et 2 (intervalles fermés c'est à dire comprenant les bornes) ;
où m représente zéro ou un entier entre 1 et 2 (intervalles fermés c'est à dire comprenant les bornes) ;
où X et X', semblables ou différents, représente un bras comportant au plus deux chaînons carbonés ;
où s est zéro ou un entier choisi entre 1 et 30 ;
où n est zéro ou un entier choisi entre 1 et 30,

avec la condition que lorsque s et/ou n sont égaux à zéro, E est phosphore et que lorsque s et n sont égaux à zéro, $R_1$ et/ou $R_2$ sont alcoyles en $C_8$-$C_{12}$ ou aralcoyle en $C_{12}$-$C_{16}$ ou alcoylaryle en $C_{10}$-$C_{14}$ ;

où E est un élément choisi parmi le carbone et les éléments métalloïdes de rang atomique au moins égal à celui du phosphore et appartenant à la colonne VB ou aux chalcogènes de rang atomique au moins égal à celui du soufre ;

où $R_1$ et $R_2$, semblables ou différents, représentent un radical hydrocarboné, éventuellement substitué ;
où $M^+$ est un contre-cation,
ou ladite composition comprenant

- un composé (poly)isocyanate non masqué ou partiellement masqué et au moins partiellement sous la forme

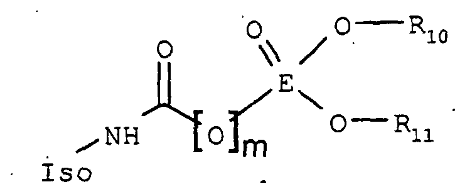

où m est le même que dans la Formule I.

⇨ où E est un élément de la colonne VA la classification périodique des éléments ;
⇨ où Iso est le reste (d'un poly)isocyanate (après élimination d'une fonction isocyanate) ;
⇨ où $R_{10}$ est une charge négative ou un reste hydrocarboné contenant des atomes d'hydrogène et de carbone lié à l'atome d'oxygène par un carbone ;
⇨ où $R_{11}$ est choisi parmi :

⇓ une charge négative ;
⇓ un groupe de formule :

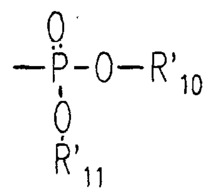

dans laquelle $R'_{10}$ est choisi parmi une charge négative et les restes hydrocarbonés semblables ou différents de $R_{10}$ et lié à l'atome d'oxygène par un carbone

dans laquelle le(s) $R'_{11}$ est (sont) choisi(s) parmi une charge négative et les restes hydrocarbonés semblables ou différents de $R_{10}$ et de $R'_{11}$ et liés par un atome d'oxygène par un atome de carbone.

sous réserve qu'au moins un des substituants $R_{10}$, $R'_{10}$ et $R'_{11}$ comporte un fragment de chaîne polyéthylène glycol d'au moins 1,

et éventuellement un composé de formule I tel que défini ci-dessus.

**14.** Emulsion ou suspension aqueuse selon la revendication 13, **caractérisée en ce qu'**elle présente les caractéristiques des revendications 2 à 11.

**15.** Emulsion ou suspension aqueuse selon l'une des revendications 13 ou 14, **caractérisée en ce que** le rapport massique entre les isocyanates à mettre en suspension et ledit composé comportant une fonction anionique est

avantageusement supérieur à 1 %.

**16.** Emulsion ou suspension aqueuse selon l'une des revendications 13 à 15, **caractérisée en ce que** la quantité dudit ou desdits composés comportant une fonction anionique correspond à une valeur comprise entre $10^{-2}$ et 1, avantageusement entre $5.10^{-2}$ et 0,5 atome de E par litre.

**17.** Emulsion ou suspension aqueuse selon l'une des revendications 13 à 16, **caractérisée en ce que** le rapport massique entre les isocyanates à mettre en suspension et ledit composé comportant une fonction anionique est avantageusement au moins égal à 2 %, et au plus égal à environ 20 %.

**18.** Emulsion ou suspension aqueuse selon l'une des revendications 13 à 17, **caractérisée en ce qu'**elle comprend également un polyol.

**19.** Emulsion ou suspension aqueuse selon la revendication 18, **caractérisée en ce que**, après mise en dispersion ou émulsion dans l'eau, la somme des constituants isocyanates, composé anionique et polyol varie de 30 à 70 % en masse par rapport à la masse de la totalité de la composition.

**20.** Emulsion ou suspension aqueuse selon l'une des revendications 18 ou 19, **caractérisée par le fait que** ledit polyol est un nanolatex.

**21.** Emulsion ou suspension aqueuse selon la revendication 20 **caractérisée par le fait que** ledit polyol est un nanolatex présentant les caractéristiques suivante $d_{50}$ compris entre 15 et 60 nm ;

fonction carboxylate de 0, 5 à 5% en masse
fonction ol :entre 1 et 3 %
taux de solide : entre 25 et 40 %
un $d_{80}$ inférieur à 1 micromètre.

**22.** Emulsion selon la revendication 13, **caractérisée par le fait que** ladite émulsion est une émulsion d'isocyanate dans l'eau qui présente un $d_{50} \leq$ (au plus égal à) à 5 micromètres.

**23.** Emulsion selon les revendications 13 à 22, **caractérisée par le fait qu'**elle constitue une émulsion de type huile dans eau de teneur en eau de 10 à 70%.

**24.** Emulsion ou suspension selon les revendications 18 à 23, **caractérisée par le fait que** teneur en isocyanate + émulsifiant + alcool dans eau 30 à 70 %.

**25.** Procédé de mise en émulsion ou suspension d'une émulsion ou suspension selon la revendication 24, **caractérisé par le fait que** comporte l'étape suivante : ajout du isocyanate dans le mélange polyol + eau.

**26.** Procédé selon la revendication 24, **caractérisé par le fait que** l'agitation est manuelle ou mécanique.

**27.** Procédé selon les revendications 25 ou 26, **caractérisé par le fait qu'**il est réalisé à température ambiante inférieure à 50°C.

**28.** Procédé selon les revendications 25 à 27, **caractérisé par le fait que** l'on ajuste le pH de la phase aqueuse à une valeur supérieur à 3 avantageusement à 4.

**29.** Procédé selon les revendications 25 à 28, **caractérisé par le fait que** les pigments sont dispersés dans le ou les polyols avant l'addition de l'isocyanate.

**30.** Procédé de préparation d'un revêtement, **caractérisé par le fait qu'**il comporte l'application d'une couche d'une émulsion ou suspension selon la revendication 13, sur une épaisseur comprise entre 10 à 400 micromètres avant séchage, et correspondant après séchage à une épaisseur comprise entre 5 et 150 micromètres.

**31.** Procédé selon la revendication 30, **caractérisé par le fait qu'**il comporte un séchage de 20°C à 60°C pendant 1/4 à 24 heures.

**32.** Procédé selon les revendications 30 à 31, **caractérisé par le fait qu'**il comporte un séchage en présence d'un solvant pour aider à l'élimination de l'eau.

**33.** Procédé selon les revendications 30 à 32, **caractérisé par le fait que** la couche est appliquée par pulvérisation, ou au moyen d'applicateur à tige filetée.

**34.** Composé de formule :

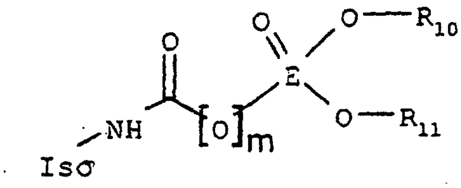

où m est la même que dans la Formule I ;

⇨ où E est un élément de la colonne VA la classification périodique des éléments ;
⇨ où Iso est le reste (d'un poly)isocyanate (après élimination d'une fonction isocyanate)
⇨ où $R_{10}$ est une charge négative ou un reste hydrocarboné, contenant des atomes d'hydrogène et de carbone lié à l'atome d'oxygène par un carbone ;
⇨ où $R_{11}$ est choisi parmi :

⇓ une charge négative ;
⇓ un groupe de formule :

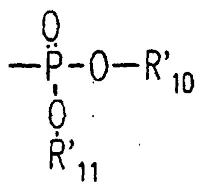

dans laquelle $R'_{10}$ est choisi parmi une charge négative et les restes hydrocarbonés semblables ou différents de $R_{10}$ lié à l'atome d'oxygène par un carbone

dans laquelle le(s) $R'_{11}$ est (sont) choisi(s) parmi une charge négative et les restes hydrocarbonés semblables ou différents de $R_{10}$ et de $R'_{11}$ liés par un atome d'oxygène par un carbone,

sous réserve que lorsque E représente le phosphore l'un de $R_{10}$, $R'_{10}$ et $R'_{11}$ comportent un fragment de chaîne polyéthylène glycol d'au moins 1 unité éthylényloxyle.

**35.** Composé selon la revendication 34, **caractérisé par le fait qu'**iso est le reste d'un trimère (isocyanurate) ou d'un biuret dont au moins un des monomères porte ses fonctions isocyanates par l'intermédiaire d'enchaînement éthylène.

**36.** Composé selon les revendications 34 ou 35, **caractérisé par le fait qu'**iso est le reste d'un trimère (isocyanurate) ou d'un biuret dont les monomères portent ses fonctions isocyanates par l'intermédiaire d'enchaînement éthylène.

**37.** Composé selon les revendications 35 ou 36, **caractérisé par le fait que** le(s) monomère portant ses fonctions isocyanates par l'intermédiaire d'enchaînement éthylène sont des polyméthylène diisocyanates avantageusement des hexaméthylène diisocyanates.

**38.** Composé selon l'une quelconque des revendications 34 à 36 **caractérisé par le fait qu'**iso est le reste d'un trimère (isocyanurate) d'hexaméthylène diisocyanate.

**39.** Utilisation d'un composé anionique de formule générale I suivante :

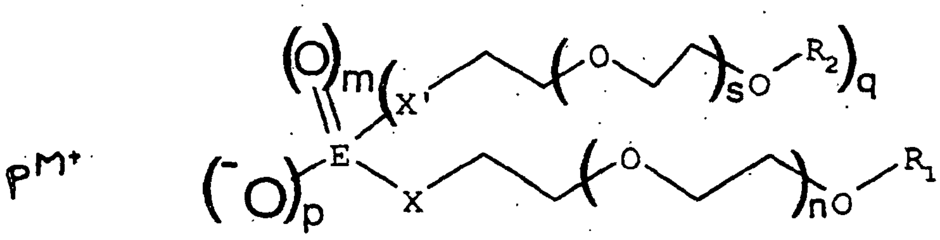

où q représente zéro ou 1 ;
où p représente un entier entre 1 et 2 (intervalles fermés c'est à dire comprenant les bornes) ;
où m représente zéro ou un entier entre 1 et 2 (intervalles fermés c'est à dire comprenant les bornes) ;
où X et X', semblables ou différents, représente un bras comportant au plus deux chaînons carbonés ;
où s est zéro ou un entier choisi entre 1 et 30 ;
où n est zéro ou un entier choisi entre 1 et 30 ;
où E est un élément choisi parmi le carbone et les éléments métalloïdes de rang atomique au moins égal à celui du phosphore et appartenant à la colonne VB ou aux chalcogènes de rang atomique au moins égal à celui du soufre ;
où $R_1$ et $R_2$, semblables ou différents, représentent un radical hydrocarboné éventuellement substitué ;
où $M^+$ est un contre-cation
sous réserve que lorsque E est le phosphore, la somme de s et n soit au moins égale à 1,
pour l'obtention d'une émulsion ou suspension aqueuse de polyisocyanate et de polyol.

**40.** Composition selon la revendication 1 ou émulsion ou suspension aqueuse selon la revendication 13, **caractérisée en ce que** au moins deux des fonctions NCO du composé isocyanate sont reliées à un squelette hydrocarboné par l'intermédiaire d'un carbone saturé ($sp^3$).

**41.** Composition selon la revendication 1 ou émulsion ou suspension aqueuse selon la revendication 16 ou utilisation selon la revendication 39 **caractérisée en ce que** s est compris entre 5 et 25.

**42.** Composition selon la revendication 1 ou émulsion ou suspension aqueuse selon la revendication 16 ou utilisation selon la revendication 39 **caractérisée en ce que** s est compris entre 9 et 20.

**43.** Composition selon la revendication 1 ou émulsion ou' suspension aqueuse selon la revendication 16 ou utilisation selon la revendication 39 **caractérisée en ce que** n est compris entre 5 et 25.

**44.** Composition selon la revendication 1 ou émulsion ou suspension aqueuse selon la revendication 16 ou utilisation selon la revendication 39 **caractérisée en ce que** n est compris entre 9 et 20.

**45.** Composition selon la revendication 1 ou émulsion ou suspension aqueuse selon la revendication 16 où utilisation selon la revendication 39, **caractérisée en ce que** $R_1$ et $R_2$ sont des groupes aryles ou alcoyles éventuellement substitués.

**46.** Composition selon la revendication 1 ou émulsion ou suspension aqueuse selon la revendication 16, **caractérisée en ce que** au moins un des substituants $R_{10}$, $R'_{10}$ et $R'_{11}$ comporte un fragment de chaîne polyéthylène glycol d'au moins 5 unités éthylényloxyles.

**47.** Composition selon la revendication 1 ou émulsion ou suspension aqueuse selon la revendication 16, **caractérisée en ce que** au moins un des substituants $R_{10}$, $R'_{10}$ et $R'_{11}$ comporte un fragment de chaîne polyéthylène glycol d'au moins 7 unités éthylényloxyles.

**48.** Composition selon la revendication 1 ou émulsion ou suspension aqueuse selon la revendication 16 ou composé selon la revendication 34 ou utilisation selon la revendication 39 **caractérisée en ce que** E représente phosphore.

**Patentansprüche**

1. Zusammensetzung auf der Grundlage von Isocyanat(en), umfassend:

- eine nichtmaskierte oder teilweise maskierte (Poly)isocyanat-Verbindung, bei der mindestens eine der NCO-Funktionen über einen gesättigten Kohlenstoff ($sp^3$) an ein Kohlenwasserstoffskelett gebunden ist; und
- ein anionisches oberflächenaktives Mittel der folgenden allgemeinen Formel I:

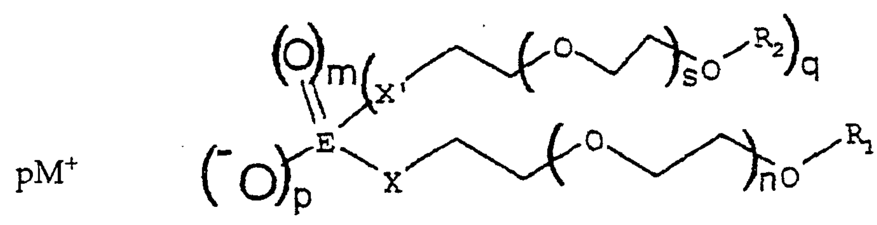

worin q Null oder 1 bedeutet;

worin p eine ganze Zahl zwischen 1 und 2 bedeutet (geschlossene Bereiche, das heißt einschließlich der Grenzwerte);

worin m Null oder eine ganze Zahl zwischen 1 und 2 bedeutet (geschlossene Bereiche, das heißt einschließlich der Grenzwerte);

worin X und X', die ähnlich oder verschieden sein können, einen Zweig, der höchstens zwei Kohlenstoff-haltige Kettenglieder aufweist, bedeuten;

worin s Null oder eine ganze Zahl ausgewählt zwischen 1 und 30 bedeutet;

worin n Null oder eine ganze Zahl ausgewählt zwischen 1 und 30 bedeutet; mit der Maßgabe, daß, wenn s und/oder n Null bedeuten E Phosphor bedeutet und wenn s und n Null bedeuten, $R_1$ und/oder $R_2$ $C_8$-$C_{12}$-Alkyl oder $C_{12}$-$C_{16}$-Aralkyl oder $C_{10}$-$C_{14}$-Alkylaryl bedeuten;

worin E ein Element ausgewählt aus Kohlenstoff und Metalloidelementen mit einer Ordnungszahl, die mindestens gleich der von Phosphor ist und die zu der Gruppe VB oder Chalkogenen mit einer Ordnungszahl, die mindestens gleich der von Schwefel ist, gehören, bedeutet;

worin $R_1$ und $R_2$, die ähnlich oder verschieden sein können, einen gegebenenfalls substituierten Kohlenwasserstoffrest bedeuten;

worin $M^+$ ein Gegenkation bedeutet,

welche Zusammensetzung frei ist von polyfunktionellen Hydroxylgruppen-haltigen Verbindungen mit einem zahlenmittleren Molekulargewicht von 400 bis 5000;

oder die Zusammensetzung umfaßt

- eine nichtmaskierte oder teilweise maskierte (Poly)isocyanat-Verbindung, bei der mindestens eine der NCO-Funktionen über einen gesättigten Kohlenstoff ($sp^3$) an ein Kohlenstoffwasserstoffskelett gebunden ist, welche mindestens teilweise in der folgenden Form vorliegt

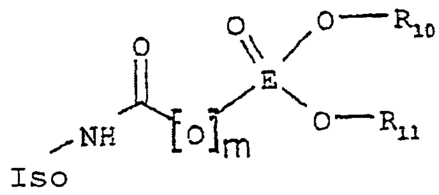

in der m die gleiche Bedeutung besitzt wie bezüglich der Formel I;

⇨ worin E ein Element der Gruppe VA des Periodensystems der Elemente ist;
⇨ worin Iso für den Rest eines (Poly)isocyanats steht;
⇨ worin $R_{10}$ für eine negative Ladung oder ein Kohlenwasserstoffrest steht, der Wasserstoff- und Kohlenstoffatome enthält und über einen Kohlenstoff an das Sauerstoffatom gebunden ist;

⇨ worin $R_{11}$ ausgewählt ist aus:

⇩ einer negativen Ladung;
⇩ einer Gruppe der Formel:

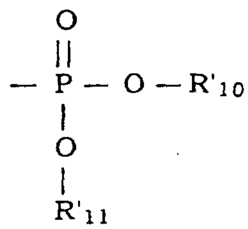

worin $R'_{10}$ ausgewählt ist aus einer negativen Ladung und Kohlenwasserstoffresten, die ähnlich oder verschieden sind von $R_{10}$ und über einen Kohlenstoff an das Sauerstoffatom gebunden ist;
worin $R'_{11}$ ausgewählt ist (sind) aus einer negativen Ladung und Kohlenwasserstoffresten, die ähnlich oder verschieden sind von $R_{10}$ und $R'_{11}$ und über ein Kohlenstoffatom an ein Sauerstoffatom gebunden ist (sind),

mit der Maßgabe, daß mindestens einer der Substituenten $R_{10}$, $R'_{10}$ und $R'_{11}$ ein Fragment einer Polyethylenglykolkette von mindestens 1, vorteilhafterweise von mindestens 5, vorzugsweise von mindestens 7 Ethylenoxideinheiten trägt,
und gegebenenfalls eine Verbindung der Formel I, wie sie oben definiert worden ist.

**2.** Zusammensetzung auf der Grundlage von Isocyanat(en) nach Anspruch 1, **dadurch gekennzeichnet, daß** sämtliche Kohlenstoffatome, über welche die Isocyanatfunktionen an das Kohlenwasserstoffskelett gebunden sind, gesättigte Kohlenstoffatome $sp^3$ sind.

**3.** Zusammensetzung nach Anspruch 1 oder 2, **dadurch gekennzeichnet, daß**:

- $R_1$ und $R_2$ ($C_8$-$C_{12}$)-Alkylgruppen, $C_{12}$-$C_{16}$-Aralkylgruppen, $C_{10}$-$C_{14}$-Alkylarylgruppen und $C_{10}$-$C_{16}$-Arylgruppen bedeuten, wenn die Zahl der Ethylenoxidfunktionen in der Formel I höchstens gleich 5 ist; oder
- $R_1$ und $R_2$ eine Kohlenwasserstoffgruppe bedeuten, die mindestens 1 und höchstens 25 Kohlenstoffatome aufweist, wenn die Zahl der Ethylenoxidfunktionen größer ist als 5.

**4.** Zusammensetzung nach einem der Ansprüche 1 bis 3, **dadurch gekennzeichnet, daß** einer der Reste X und gegebenenfalls der Reste X' aus zweiwertigen Resten ausgewählt ist, die gebildet sind durch:

- O-P(O)($O^-$)-X"-, wenn E P bedeutet;
- -O-($R_{10}$-O)P(O)-X"-, wenn E P bedeutet, wobei X" Sauerstoff oder eine Einfachbindung bedeutet;
- eine direkte Bindung zwischen E und der ersten Ethylengruppe des Fragments der Polyethylenglykolkette;
- eine gegebenenfalls substituierte Methylengruppe;
- einen Zweig der Struktur -Y- und der Struktur -D-Y-, -Y-D-;

worin Y ein Chalkogen, Metalloidelemente mit einer Ordnungszahl, die höchstens gleich der von Phosphor ist, und die der Gruppe VB angehören in Form von tertiären Amin- oder Phosphin-Derivaten;
worin D gegebenenfalls substituiertes, einschließlich funktionalisiertes Alkyl bedeutet.

**5.** Zusammensetzung nach Anspruch 4, **dadurch gekennzeichnet, daß** einer der Reste X und X' ein Rest der Formel $[EO_m(O^-)_p]$ ist, worin m und p die oben angegebenen Bedeutungen besitzen.

**6.** Zusammensetzung nach einem der vorhergehenden Ansprüche, **dadurch gekennzeichnet, daß** E aus Kohlenstoff, Phosphor und Schwefel ausgewählt ist.

**7.** Zusammensetzung nach Anspruch 6, **dadurch gekennzeichnet, daß** E Phosphor ist.

**8.** Zusammensetzung nach einem der vorhergehenden Ansprüche, **dadurch gekennzeichnet, daß** die anionische Verbindung der Formel II entspricht:

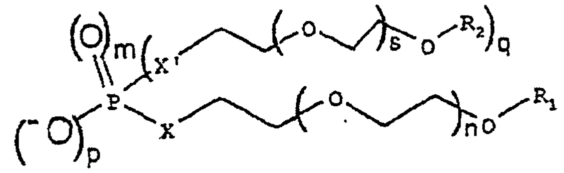

und wenn q Null bedeutet:

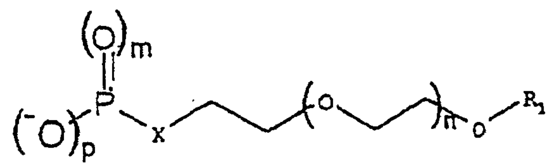

- worin p Null oder eine ganze Zahl zwischen 1 und 2 bedeutet;
- worin m Null oder eine ganze Zahl zwischen 1 und 2 bedeutet;
- worin die Summe p + m + q höchstens gleich Drei ist;
- worin die Summe 1 + p + 2m + q gleich Drei oder Fünf ist;
- worin X und X', die ähnlich oder verschieden sind, einen Zweig bedeuten, der höchstens zwei Kohlenstoff-haltige Kettenglieder aufweist;
- worin n und s, die ähnlich oder verschieden sind, eine ganze Zahl bedeuten ausgewählt zwischen 5 und 30;
- worin $R_1$ und $R_2$, die ähnlich oder verschieden sind, einen gegebenenfalls substituierten Kohlenwasserstoffrest bedeuten.

**9.** Zusammensetzung nach Anspruch 1, **dadurch gekennzeichnet, daß**, wenn E Phosphor bedeutet, die anionische Verbindung aus einer Monoester/Diester-Mischung besteht in einem Molverhältnis zwischen 1/10 und 10, welche zusätzlich 1 % bis etwa 20 Massen-% Phosphorsäure und 0 bis 5 % Pyrophosphorsäureester enthalten kann.

**10.** Zusammensetzung nach Anspruch 1, **dadurch gekennzeichnet, daß** das Massenverhältnis zwischen den oberflächenaktiven Verbindungen und den Isocyanaten zwischen 4 und 10 % liegt.

**11.** Zusammensetzung nach den vorhergehenden Ansprüchen, **dadurch gekennzeichnet, daß** das Gegenkation ein vorzugsweise tertiäres Amin ist.

**12.** Zusammensetzung nach den Ansprüchen 1 bis 10, **dadurch gekennzeichnet, daß** die Zusammensetzung zusätzlich einen vorzugsweise latenten Katalysator umfaßt.

**13.** Wäßrige Emulsion oder Suspension umfassend:

- eine nichtmaskierte oder teilweise maskierte (Poly)isocyanat-Verbindung, bei der mindestens eine der NCO-Funktionen über einen gesättigten Kohlenstoff ($sp^3$) an ein Kohlenwasserstoffskelett gebunden ist; und
- ein oberflächenaktives Mittel der folgenden allgemeinen Formel I:

$pM^+$ 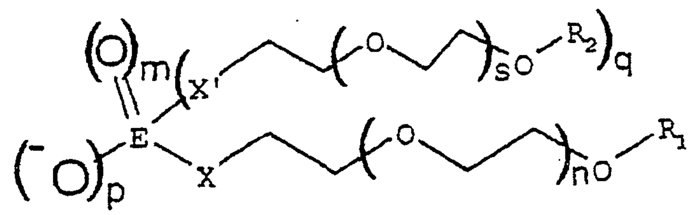

worin q Null oder 1 bedeutet;

worin p eine ganze Zahl zwischen 1 und 2 bedeutet (geschlossene Bereiche, das heißt einschließlich der Grenzwerte);

worin m Null oder eine ganze Zahl zwischen 1 und 2 bedeutet (geschlossene Bereiche, das heißt einschließlich der Grenzwerte);

worin X und X', die ähnlich oder verschieden sein können, einen Zweig, der höchstens zwei Kohlenstoff-haltige Kettenglieder aufweist, bedeuten;

worin s Null oder eine ganze Zahl ausgewählt zwischen 1 und 30 bedeutet;

worin n Null oder eine ganze Zahl ausgewählt zwischen 1 und 30 bedeutet; mit der Maßgabe, daß, wenn s und/oder n Null bedeuten E Phosphor bedeutet und wenn s und n Null bedeuten, $R_1$ und/oder $R_2$ $C_8$-$C_{12}$-Alkyl oder $C_{12}$-$C_{16}$-Aralkyl oder $C_{10}$-$C_{14}$-Alkylaryl bedeuten;

worin E ein Element ausgewählt aus Kohlenstoff und Metalloidelementen mit einer Ordnungszahl, die mindestens gleich der von Phosphor ist und die zu der Gruppe VB oder Chalkogenen mit einer Ordnungszahl, die mindestens gleich der von Schwefel ist, gehören, bedeutet;

worin $R_1$ und $R_2$, die ähnlich oder verschieden sein können, einen gegebenenfalls substituierten Kohlenwasserstoffrest bedeuten;

worin $M^+$ ein Gegenkation bedeutet;

oder die Zusammensetzung umfaßt

- eine nichtmaskierte oder teilweise maskierte (Poly)isocyanat-Verbindung, welche mindestens teilweise in der folgenden Form vorliegt

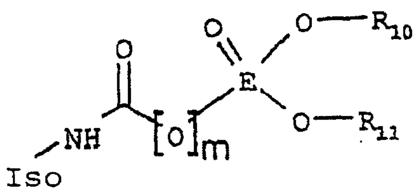

in der m die gleiche Bedeutung besitzt wie bezüglich der Formel I;

⇨ worin E ein Element der Gruppe VA des Periodensystems der Elemente ist;

⇨ worin Iso für den Rest eines (Poly)isocyanats steht;

⇨ worin $R_{10}$ für eine negative Ladung oder ein Kohlenwasserstoffrest steht, der Wasserstoff- und Kohlenstoffatome enthält und über einen Kohlenstoff an das Sauerstoffatom gebunden ist;

⇨ worin $R_{11}$ ausgewählt ist aus:

⇩ einer negativen Ladung;

⇩ einer Gruppe der Formel:

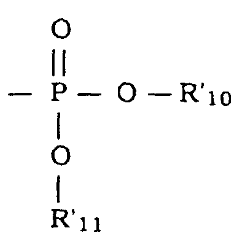

worin $R'_{10}$ ausgewählt ist aus einer negativen Ladung und Kohlenwasserstoffresten, die ähnlich oder verschieden sind von $R_{10}$ und über einen Kohlenstoff an das Sauerstoffatom gebunden ist;

worin $R'_{11}$ ausgewählt ist (sind) aus einer negativen Ladung und Kohlenwasserstoffresten, die ähnlich oder verschieden sind von $R_{10}$ und $R'_{11}$ und über ein Kohlenstoffatom an ein Sauerstoffatom gebunden ist (sind),

mit der Maßgabe, daß mindestens einer der Substituenten $R_{10}$, $R'_{10}$ und $R'_{11}$ ein Fragment einer Polyethy-

lenglykolkette von mindestens 1 trägt,
und gegebenenfalls eine Verbindung der Formel I, wie sie oben definiert worden ist.

**14.** Wäßrige Emulsion oder Suspension nach Anspruch 13, **dadurch gekennzeichnet, daß** sie die Kennzeichen der Ansprüche 2 bis 11 aufweist.

**15.** Wäßrige Emulsion oder Suspension nach einem der Ansprüche 13 oder 14, **dadurch gekennzeichnet, daß** das Massenverhältnis zwischen den zu suspendierenden Isocyanaten und der eine anionische Funktion aufweisenden Verbindung mit Vorteil größer ist als 1 %.

**16.** Wäßrige Emulsion oder Suspension nach einem der Ansprüche 13 bis 15, **dadurch gekennzeichnet, daß** die Menge der Verbindung(en), welche eine anionische Funktion aufweist (aufweisen) einem Wert zwischen $10^{-2}$ und 1, vorzugsweise zwischen $5 \cdot 10^{-2}$ und 0,5 Atomen von E pro Liter entspricht.

**17.** Wäßrige Emulsion oder Suspension nach einem der Ansprüche 13 bis 16, **dadurch gekennzeichnet, daß** das Massenverhältnis zwischen den zu suspendierenden Isocyanaten und der eine anionische Funktion aufweisenden Verbindung mit Vorteil mindestens 2 % und höchstens etwa 20 % beträgt.

**18.** Wäßrige Emulsion oder Suspension nach einem der Ansprüche 13 bis 17, **dadurch gekennzeichnet, daß** sie zusätzlich ein Polyol umfaßt.

**19.** Wäßrige Emulsion oder Suspension nach Anspruch 18, **dadurch gekennzeichnet, daß** nach dem Dispergieren oder Emulgieren in Wasser die Summe der Isocyanat-Bestandteile, der anionischen Verbindung und Polyol zwischen 30 und 70 Massen-%, bezogen auf die Masse der Gesamtheit der Zusammensetzung, variiert.

**20.** Wäßrige Emulsion oder Suspension nach einem der Ansprüche 18 oder 19, **dadurch gekennzeichnet, daß** das Polyol ein Nanolatex ist.

**21.** Wäßrige Emulsion oder Suspension nach Anspruch 20, **dadurch gekennzeichnet, daß** das Polyol ein Nanolatex ist, der die folgenden Eigenschaften besitzt:

$d_{50}$ zwischen 15 und 60 nm;
Carboxylatfunktion 0,5 bis 5 Massen-%
Ol-Funktion: zwischen 1 und 3 %
Feststoffgehalt: zwischen 25 und 40 %
$d_{80}$ weniger als 1 Mikrometer.

**22.** Emulsion nach Anspruch 13, **dadurch gekennzeichnet, daß** die Emulsion eine Emulsion von Isocyanat in Wasser ist, welche einen $d_{50}$-Wert $\leq$ (höchstens gleich) 5 Mikrometer aufweist.

**23.** Emulsion nach den Ansprüchen 13 bis 22, **dadurch gekennzeichnet, daß** sie eine Emulsion vom Typ Öl-in-Wasser mit einem Wassergehalt von 10 bis 70 % ist.

**24.** Emulsion oder Suspension nach den Ansprüchen 18 bis 23, **dadurch gekennzeichnet, daß** der Gehalt an Isocyanat + Emulgator + Alkohol in Wasser 30 bis 70 % beträgt.

**25.** Verfahren zum Emulgieren oder Suspendieren einer Emulsion oder Suspension nach Anspruch 24, **dadurch gekennzeichnet, daß** sie die folgende Stufe umfaßt: Zugabe des Isocyanats zu der Mischung aus Polyol + Wasser.

**26.** Verfahren nach Anspruch 24, **dadurch gekennzeichnet, daß** das Rühren manuell oder mechanisch erfolgt.

**27.** Verfahren nach den Ansprüchen 25 oder 26, **dadurch gekennzeichnet, daß** es bei einer Umgebungstemperatur unterhalb 50°C durchgeführt wird.

**28.** Verfahren nach den Ansprüchen 25 bis 27, **dadurch gekennzeichnet, daß** man den pH-Wert der wäßrigen Phase auf einen Wert von mehr als 3, mit Vorteil von 4, einstellt.

**29.** Verfahren nach den Ansprüchen 25 bis 28, **dadurch gekennzeichnet, daß** die Pigmente vor der Zugabe des

Isocyanats in dem oder den Polyolen dispergiert werden.

**30.** Verfahren zur Herstellung eines Überzugs, **dadurch gekennzeichnet, daß** es das Aufbringen einer Schicht einer Emulsion oder Suspension nach Anspruch 13 mit einer Dicke zwischen 10 bis 400 Mikrometer vor dem Trocknen und entsprechend einer Dicke zwischen 5 und 150 Mikrometer nach dem Trocknen umfaßt.

**31.** Verfahren nach Anspruch 30, **dadurch gekennzeichnet, daß** es einen Trocknungsvorgang bei 20°C bis 60°C während 1/4 bis 24 Stunden umfaßt.

**32.** Verfahren nach den Ansprüchen 30 bis 31, **dadurch gekennzeichnet, daß** es einen Trocknungsvorgang in Gegenwart eines Lösungsmittels zur Unterstützung der Beseitigung des Wassers umfaßt.

**33.** Verfahren nach den Ansprüchen 30 bis 32, **dadurch gekennzeichnet, daß** die Schicht durch Aufsprühen oder mit Hilfe einer Gewindestangen-Auftragsvorrichtung aufgebracht wird.

**34.** Verbindung der Formel:

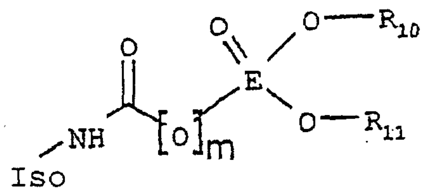

worin m die gleiche Bedeutung besitzt wie bezüglich der Formel I;

⇨ worin E ein Element der Gruppe VA des Periodensystems der Elemente ist;
⇨ worin Iso für den Rest eines (Poly)isocyanats steht (nach der Abspaltung einer Isocyanatfunktion);
⇨ worin $R_{10}$ für eine negative Ladung oder ein Kohlenwasserstoffrest steht, der Wasserstoff- und Kohlenstoffatome aufweist und über einen Kohlenstoff an ein Sauerstoffatom gebunden ist;
⇨ worin $R_{11}$ ausgewählt ist aus:

⇩ einer negativen Ladung;
⇩ einer Gruppe der Formel:

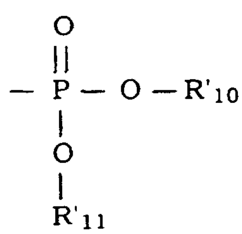

worin $R'_{10}$ ausgewählt ist aus einer negativen Ladung und Kohlenwasserstoffresten, die ähnlich oder verschieden sind von $R_{10}$ und über einen Kohlenstoff an das Sauerstoffatom gebunden ist;
worin $R'_{11}$ ausgewählt ist (sind) aus einer negativen Ladung und Kohlenwasserstoffresten, die ähnlich oder verschieden sind von $R_{10}$ und $R'_{11}$ und über ein Kohlenstoffatom an ein Sauerstoffatom gebunden ist (sind),

mit der Maßgabe, daß, wenn E Phosphor bedeutet, einer der Reste $R_{10}$, $R'_{10}$ und $R'_{11}$ ein Fragment einer Polyethylenglykolkette mit mindestens einer Ethylenoxideinheit umfaßt.

**35.** Verbindung nach Anspruch 34, **dadurch gekennzeichnet, daß** Iso der, Rest eines Trimeren (Isocyanurat) oder Biurets ist, von dem mindestens eines der Monomeren seine Isocyanatfunktionen über eine Ethylenkette trägt.

**36.** Verbindung nach den Ansprüchen 34 oder 35, **dadurch gekennzeichnet, daß** Iso der Rest eines Trimeren (Isocyanurat) oder Biurets ist, deren Monomeren ihre Isocyanatfunktionen über eine Ethylenkette tragen.

**37.** Verbindung nach den Ansprüchen 35 oder 36, **dadurch gekennzeichnet, daß** das (die) Monomere(n), welche(s) seine (ihre) Isocyanatfunktionen über eine Ethylenkette tragen, Polymethylendiisocyanate, vorzugsweise Hexamethylendiisocyanate, sind.

**38.** Verbindung nach einem der Ansprüche 34 bis 36, **dadurch gekennzeichnet, daß** Iso den Rest eines Trimeren (Isocyanurat) von Hexamethylendiisocyanat bedeutet.

**39.** Verwendung einer anionischen Verbindung der folgenden allgemeinen Formel I:

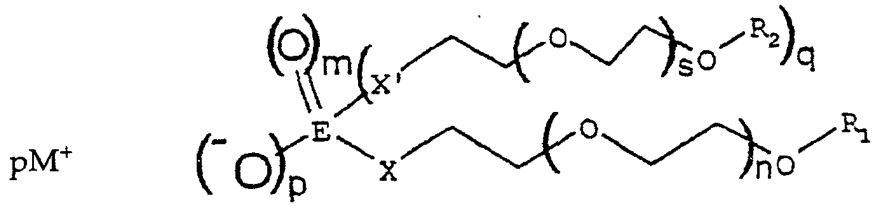

worin q Null oder 1 bedeutet;

worin p eine ganze Zahl zwischen 1 und 2 bedeutet (geschlossene Bereiche, das heißt einschließlich der Grenzwerte);

worin m Null oder eine ganze Zahl zwischen 1 und 2 bedeutet (geschlossene Bereiche, das heißt einschließlich der Grenzwerte);

worin X und X', die ähnlich oder verschieden sein können, einen Zweig, der höchstens zwei Kohlenstoff-haltige Kettenglieder aufweist, bedeuten;

worin s Null oder eine ganze Zahl ausgewählt zwischen 1 und 30 bedeutet;

worin n Null oder eine ganze Zahl ausgewählt zwischen 1 und 30 bedeutet;

worin E ein Element ausgewählt aus Kohlenstoff und Metalloidelementen mit einer Ordnungszahl, die mindestens gleich der von Phosphor ist und die zu der Gruppe VB oder Chalkogenen mit einer Ordnungszahl, die mindestens gleich der von Schwefel ist, gehören, bedeutet;

worin $R_1$ und $R_2$, die ähnlich oder verschieden sein können, einen gegebenenfalls substituierten Kohlenwasserstoffrest bedeuten;

worin $M^+$ ein Gegenkation bedeutet,

mit der Maßgabe, daß, wenn E Phosphor bedeutet, die Summe von s und n mindestens gleich 1 ist,

für die Herstellung einer wäßrigen Emulsion oder Suspension von Polyisocyanat und Polyol.

**40.** Zusammensetzung nach Anspruch 1 oder wäßrige Emulsion oder Suspension nach Anspruch 13, **dadurch gekennzeichnet, daß** mindestens zwei der NCO-Funktionen der Isocyanatverbindung über einen gesättigten Kohlenstoff ($sp^3$) an ein Kohlenwasserstoffskelett gebunden sind.

**41.** Zusammensetzung nach Anspruch 1 oder wäßrige Emulsion oder Suspension nach Anspruch 16 oder Verwendung nach Anspruch 39, **dadurch gekennzeichnet, daß** s einen Wert zwischen 5 und 25 aufweist.

**42.** Zusammensetzung nach Anspruch 1 oder wäßrige Emulsion oder Suspension nach Anspruch 16 oder Verwendung nach Anspruch 39, **dadurch gekennzeichnet, daß** s einen Wert zwischen 9 und 20 aufweist.

**43.** Zusammensetzung nach Anspruch 1 oder wäßrige Emulsion oder Suspension nach Anspruch 16 oder Verwendung nach Anspruch 39, **dadurch gekennzeichnet, daß** n einen Wert zwischen 5 und 25 aufweist.

**44.** Zusammensetzung nach Anspruch 1 oder wäßrige Emulsion oder Suspension nach Anspruch 16 oder Verwendung nach Anspruch 39, **dadurch gekennzeichnet, daß** n einen Wert zwischen 9 und 20 aufweist.

**45.** Zusammensetzung nach Anspruch 1 oder wäßrige Emulsion oder Suspension nach Anspruch 16 oder Verwendung nach Anspruch 39, **dadurch gekennzeichnet, daß** $R_1$ und $R_2$ gegebenenfalls substituierte Arylgruppen oder Alkylgruppen sind.

**46.** Zusammensetzung nach Anspruch 1 oder wäßrige Emulsion oder Suspension nach Anspruch 16, **dadurch gekennzeichnet, daß** mindestens einer der Substituenten $R_{10}$, $R'_{10}$ und $R'_{11}$ ein Fragment einer Polyethylenglykolkette mit mindestens 5 Ethylenoxideinheiten umfaßt.

**47.** Zusammensetzung nach Anspruch 1 oder wäßrige Emulsion oder Suspension nach Anspruch 16, **dadurch gekennzeichnet, daß** mindestens einer der Substituenten $R_{10}$, $R'_{10}$ und $R'_{11}$ ein Fragment einer Polyethylenglykolkette mit mindestens 7 Ethylenoxideinheiten umfaßt.

**48.** Zusammensetzung nach Anspruch 1 oder wäßrige Emulsion oder Suspension nach Anspruch 16 oder Verbindung nach Anspruch 34 oder Verwendung nach Anspruch 39, **dadurch gekennzeichnet, daß** E Phosphor bedeutet.

## Claims

**1.** Composition based on isocyanate(s), comprising:

- an unmasked or partially masked (poly)isocyanate compound wherein at least one of the NCO functions is attached to a hydrocarbon skeleton via a saturated carbon ($sp^3$); and
- an anionic surfactant of the following general formula I:

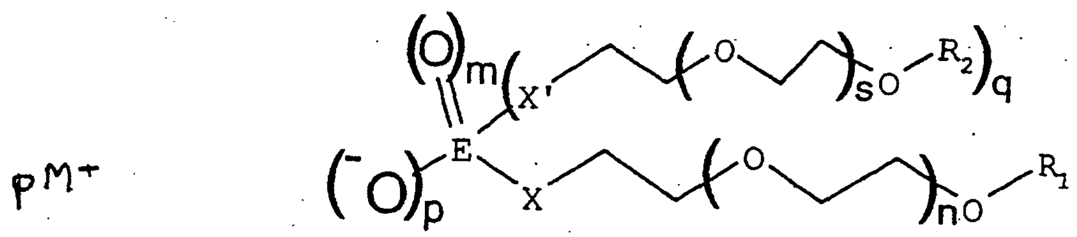

wherein q denotes zero or 1;
wherein p denotes an integer between 1 and 2 (closed intervals, i.e. inclusive of the limits);
wherein m denotes zero or an integer between 1 and 2 (closed intervals, i.e. inclusive of limits);
wherein X and X', which may be identical or different, denote an arm having not more than two carbon groups;
wherein s is zero or an integer between 1 and 30;
wherein n is zero or an integer between 1 and 30,
with the proviso that when s and/or n are equal to zero, E is phosphorus and when s and n are equal to zero, $R_1$ and/or $R_2$ are $C_{8-12}$ alkyl or $C_{12-16}$ aralkyl or $C_{10-14}$ alkylaryl;
wherein E is an element chosen from carbon and the metalloid elements of atomic rank at least equal to that of phosphorus and belonging to the column VB or the chalcogens of atomic rank at least equal to that of sulphur;
wherein $R_1$ and $R_2$ which may be identical or different denote an optionally substituted hydrocarbon radical;
wherein $M^+$ is a counter-cation,
said composition being free from polyfunctional hydroxyl compounds with a numerical mean molecular weight of from 400 to 5000;
or said composition comprising

- an unmasked or partially masked (poly)isocyanate compound wherein at least one of the NCO functions is attached to a hydrocarbon skeleton via a saturated carbon ($sp^3$) at least partially in the form

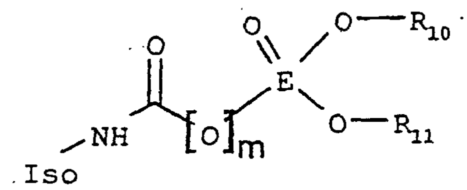

wherein m is the same as in formula I

wherein E is an element of the column VA of the Periodic Table of Elements;

wherein Iso is the residue of a (poly)isocyanate;

wherein $R_{10}$ is a negative charge or a hydrocarbon group containing hydrogen and carbon atoms linked to the oxygen atom via a carbon;

wherein $R_{11}$ is selected from among:

a negative charge;

a group of formula:

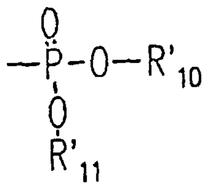

wherein $R'_{10}$ is selected from among a negative charge and the identical or different hydrocarbon groups of $R_{10}$ and is attached to the oxygen atom by a carbon

wherein the group or groups $R'_{11}$ is or are selected from among a negative charge and the identical or different hydrocarbon groups of $R_{10}$ and $R'_{11}$ and attached by an oxygen atom by a carbon atom [*sic*]

with the proviso that at least one of the substituents $R_{10}$, $R'_{10}$ and $R'_{11}$ comprises a fragment of a polyethylene glycol chain of at least 1, advantageously of at least 5, preferably of at least 7 ethylene oxide units,

and optionally a compound of formula I as hereinbefore defined.

**2.** Composition based on isocyanate(s) according to claim 1, **characterised in that** all the carbon atoms by means of which the isocyanate functions are attached to the hydrocarbon skeleton are saturated carbon atoms $sp^3$.

**3.** Composition according to claim 1 or 2, **characterised in that**:

- $R_1$ and $R_2$ denote ($C_{8-12}$) alkyl groups, $C_{12-16}$ aralkyl groups, $C_{10-14}$ alkylaryl groups and $C_{10-16}$ aryl groups, when the number of ethylene oxide functions in formula I is not more than 5; or
- $R_1$ and $R_2$ denote a hydrocarbon group having at least 1 and not more than 25 carbon atoms, when the number of ethylene oxide functions is more than 5.

**4.** Composition according to any one of claims 1 to 3, **characterised in that** one of the groups X and possibly X' are selected from among the divalent radicals consisting of:

- O-P(O)($O^-$)-X"-, when E is P;
- -O-($R_{10}$-O)P(O)-X"-, when E is P, X" denoting an oxygen or a single bond;
- a direct link between E and the first ethylene of said polyethylene glycol chain fragment;
- an optionally substituted methylene group;
- an arm of the structure -Y- and of the structure -D-Y-, -Y-D-;

where Y denotes a chalcogen, the metalloid elements of the atomic ranks at least equal to that of phosphorus and belonging to the column VB in the form of amine derivatives or tertiary phosphines;

wherein D denotes an optionally substituted, including functionalised, alkylene.

**5.** Composition according to claim 4, **characterised in that** one of the groups X and X' is a radical of formula [$EO_m(O^-)_p$], m and p being defined as hereinbefore.

**6.** Composition according to one of the preceding claims, **characterised in that** E is selected from among carbon, phosphorus and sulphur.

**7.** Composition according to claim 6, **characterised in that** E is phosphorus.

**8.** Composition according to any one of the preceding claims, **characterised in that** the anionic compound is represented by formula II:

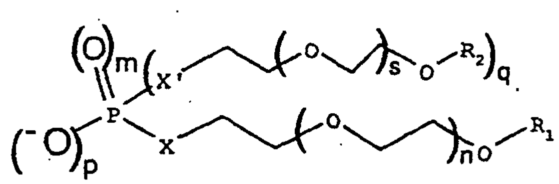

and when q is zero:

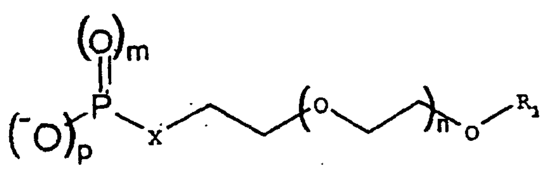

- where p denotes zero or an integer between 1 and 2;
- where m denotes zero or an integer between 1 and 2;
- where the sum of p + m + q is at most equal to three;
- where the sum of 1 + p + 2m + q is equal to three or five;
- where X and X', which may be identical or different, denote an arm having at most two carbon groups;
- where n and s, which may be identical or different, denote an integer selected between 5 and 30;
- where $R_1$ and $R_2$, which may be identical or different, represent an optionally substituted hydrocarbon group.

**9.** Composition according to claim 1, **characterised in that** when E is phosphorus the anionic compound consists of a mixture of monoester and diester in a molar ratio of between 1/10 and 10, which may further comprise from 1% to about 20% by mass of phosphoric acid and from 0 to 5% of pyrophosphoric acid esters.

**10.** Composition according to claim 1, **characterised in that** the mass ratio between the surfactant compounds and the isocyanates is between 4 and 10%.

**11.** Composition according to the preceding claims, **characterised in that** the counter-cation is an amine, advantageously a tertiary amine.

**12.** Composition according to claims 1 to 10, **characterised in that** the composition further comprises an advantageously latent catalyst.

**13.** Emulsion or aqueous suspension comprising:

- an unmasked or partially masked (poly)isocyanate compound wherein at least one of the NCO functions is attached to a hydrocarbon skeleton via a saturated carbon ($sp^3$); and
- an anionic surfactant of the following formula I:

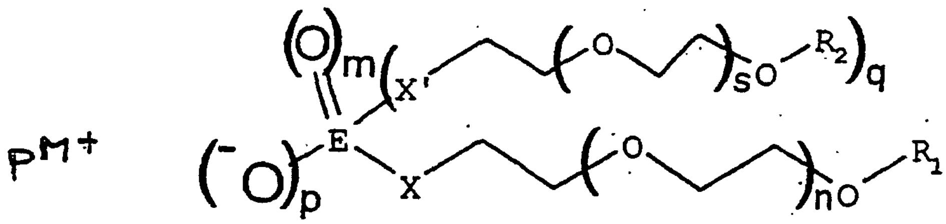

wherein q denotes zero or 1;
wherein p denotes an integer between 1 and 2 (closed intervals, i.e. inclusive of the limits);
wherein m denotes zero or an integer between 1 and 2 (closed intervals, i.e. inclusive of limits);
wherein X and X', which may be identical or different, denote an arm having not more than two carbon groups;
wherein s is zero or an integer between 1 and 30;
wherein n is zero or an integer between 1 and 30,
with the proviso that when s and/or n are equal to zero, E is phosphorus and when s and n are equal to zero, $R_1$ and/or $R_2$ are $C_{8-12}$ alkyl or $C_{12-16}$ aralkyl or $C_{10-14}$ alkylaryl;
wherein E is an element chosen from carbon and the metalloid elements of atomic rank at least equal to that of phosphorus and belonging to the column VB or the chalcogens of atomic rank at least equal to that of sulphur;
wherein $R_1$ and $R_2$ which may be identical or different denote an optionally substituted hydrocarbon radical;
wherein $M^+$ is a counter-cation,
or said composition comprising

- an unmasked or partially masked (poly)isocyanate compound at least partially in the form

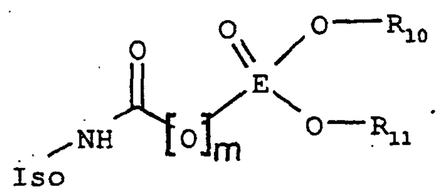

wherein m is the same as in formula I
wherein E is an element of the column VA of the Periodic Table of Elements;
wherein Iso is the residue of a (poly)isocyanate (after elimination of an isocyanate function);
wherein $R_{10}$ is a negative charge or a hydrocarbon group containing hydrogen and carbon atoms linked to the oxygen atom via a carbon;
where $R_{11}$ is selected from among:

a negative charge;
a group of formula:

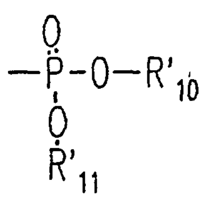

wherein $R'_{10}$ is selected from among a negative charge and the identical or different hydrocarbon groups of $R_{10}$ and is attached to the oxygen atom by a carbon
wherein the group or groups $R'_{11}$ is or are selected from among a negative charge and the identical or different

hydrocarbon groups of $R_{10}$ and $R'_{11}$ and attached by an oxygen atom by a carbon atom [*sic*]

with the proviso that at least one of the substituents $R_{10}$, $R'_{10}$ and $R'_{11}$ comprises a polyethylene glycol chain fragment of at least 1,

and optionally a compound of formula I as hereinbefore defined.

**14.** Aqueous emulsion or suspension according to claim 13, **characterised in that** it has the features of claim 2 to 11.

**15.** Aqueous emulsion or suspension according to one of claims 13 or 14, **characterised in that** the mass ratio between the isocyanates to be put into suspension and said compound comprising an anionic function is advantageously greater than 1%.

**16.** Aqueous emulsion or suspension according to one of claims 13 to 15, **characterised in that** the quantity of said compound or compounds comprising an anionic function corresponds to a value between $10^{-2}$ and 1, advantageously between $5.10^{-2}$ and 0.5 atoms of E per litre.

**17.** Aqueous emulsion or suspension according to one of claims 13 to 16, **characterised in that** the mass ratio between the isocyanates to be put into suspension and said compound comprising an anionic function is advantageously at least equal to 2% and at most equal to about 20%.

**18.** Aqueous emulsion or suspension according to one of claims 13 to 17, **characterised in that** it further comprises a polyol.

**19.** Aqueous emulsion or suspension according to claim 18, **characterised in that**, after dispersion or emulsion in water, the sum of the constituent isocyanates, anionic compound and polyol varies from 30 to 70% by mass based on the mass of the composition as a whole.

**20.** Aqueous emulsion or suspension according to one of claims 18 or 19, **characterised in that** said polyol is a nanolatex.

**21.** Aqueous emulsion or suspension according to claim, 20, **characterised in that** said polyol is a nanolatex having the following characteristics

$d_{50}$ between 15 and 60 nm;
a carboxylate function of 0.5 to 5% by mass
an ol function of between 1 and 3%
a solid content of between 25 and 40%
a $d_{80}$ of less than 1 micron.

**22.** Emulsion according to claim 13, **characterised in that** said emulsion is an emulsion of isocyanate in water which has a $d_{50} \leq$ (not more than) 5 microns.

**23.** Emulsion according to claims 13 to 22, **characterised in that** it constitutes an oil in water-type emulsion with a water content of 10 to 70%.

**24.** Emulsion or suspension according to claims 18 to 23, **characterised in that** the isocyanate + emulsifier + alcohol content in water is 30 to 70%.

**25.** Process for emulsifying or suspending an emulsion or suspension according to claim 24, **characterised in that** it comprises the following step: adding the isocyanate to the mixture of polyol + water.

**26.** Process according to claim 24, **characterised in that** the stirring is manual or mechanical.

**27.** Process according to claim 25 or 26, **characterised in that** it is carried out at ambient temperature below 50°C.

**28.** Process according to claims 25 to 27, **characterised in that** the pH of the aqueous phase is adjusted to a value greater than 3 and advantageously greater than 4.

**29.** Process according to claims 25 to 28, **characterised in that** the pigments are dispersed in the polyol or polyols

before the addition of the isocyanate.

**30.** Process for preparing a coating, **characterised in that** it comprises applying a layer of an emulsion or suspension according to claim 13 to a thickness of between 10 and 400 microns before drying and corresponding after drying to a thickness of between 5 and 150 microns.

**31.** Process according to claim 30, **characterised in that** it comprises drying at 20°C to 60°C for 1/4 hour to 24 hours.

**32.** Process according to claims 30 to 31, **characterised in that** it comprises drying in the presence of a solvent to aid the elimination of the water.

**33.** Process according to claims 30 to 32, **characterised in that** the layer is applied by spraying or by means of an applicator with a threaded rod.

**34.** Compound of formula:

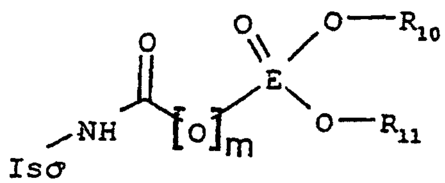

wherein m is the same as in formula I

wherein E is an element of the column VA of the Periodic Table of Elements;

wherein Iso is the residue of a (poly)isocyanate (after elimination of an isocyanate function);

wherein $R_{10}$ is a negative charge or a hydrocarbon group containing hydrogen and carbon atoms linked to the oxygen atom via a carbon;

where $R_{11}$ is selected from among:

a negative charge;

a group of formula:

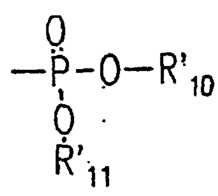

wherein $R'_{10}$ is selected from among a negative charge and the identical or different hydrocarbon groups of $R_{10}$ and is attached to the oxygen atom by a carbon

wherein the group or groups $R'_{11}$ is or are selected from among a negative charge and the identical or different hydrocarbon groups of $R_{10}$ and $R'_{11}$ and attached by an oxygen atom by a carbon atom [*sic*]

with the proviso that when E denotes phosphorus, one of $R_{10}$, $R'_{10}$ and $R'_{11}$ comprises a fragment of a polyethylene glycol chain of at least 1 ethylene oxide unit.

**35.** Compound according to claim 34, **characterised in that** iso is the residue of a trimer (isocyanurate) or a biuret at least one monomer of which carries its isocyanate functions via an ethylene chain.

**36.** Compound according to claim 34 or 35, **characterised in that** iso is the residue of a trimer (isocyanurate) or a biuret the monomers of which carry their isocyanate functions via the ethylene chain.

**37.** Compound according to claim 35 or 36, **characterised in that** the monomer or monomers carrying their isocyanate functions via ethylene chains is or are polymethylene diisocyanates, advantageously hexamethylene diisocyanates.

**38.** Compound according to any one of claims 34 to 36, **characterised in that** iso is the residue of a trimer (isocyanurate) of hexamethylene diisocyanate.

**39.** Use of an anionic compound of the following general formula I:

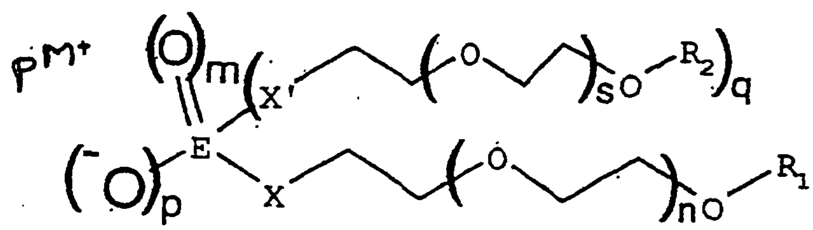

wherein q denotes zero or 1;
wherein p denotes an integer between 1 and 2 (closed intervals, i.e. inclusive of the limits);
wherein m denotes zero or an integer between 1 and 2 (closed intervals, i.e. inclusive of limits);
wherein X and X', which may be identical or different, denote an arm having not more than two carbon groups;
wherein s is zero or an integer between 1 and 30;
wherein n is zero or an integer between 1 and 30;
wherein E is an element chosen from carbon and the metalloid elements of atomic rank at least equal to that of phosphorus and belonging to the column VB or the chalcogens of atomic rank at least equal to that of sulphur;
wherein $R_1$ and $R_2$ which may be identical or different denote an optionally substituted hydrocarbon radical;
wherein $M^+$ is a counter-cation
with the proviso that when E is phosphorus the sum of s and n is at least 1,
in order to obtain an aqueous emulsion or suspension of polyisocyanate and polyol.

**40.** Composition according to claim 1 or aqueous emulsion or suspension according to claim 13, **characterised in that** at least two of the NCO functions of the isocyanate compound are attached to a hydrocarbon skeleton via a saturated carbon ($sp^3$).

**41.** Composition according to claim 1 or aqueous emulsion or suspension according to claim 16 or use according to claim 39, **characterised in that** s is between 5 and 25.

**42.** Composition according to claim 1 or aqueous emulsion or suspension according to claim 16 or use according to claim 39, **characterised in that** s is between 9 and 20.

**43.** Composition according to claim 1 or aqueous emulsion or suspension according to claim 16 or use according to claim 39, **characterised in that** n is between 5 and 25.

**44.** Composition according to claim 1 or aqueous emulsion or suspension according to claim 16 or use according to claim 39, **characterised in that** n is between 9 and 20.

**45.** Composition according to claim 1 or aqueous emulsion or suspension according to claim 16 or use according to claim 39, **characterised in that** $R_1$ and $R_2$ are optionally substituted aryl or alkyl groups.

**46.** Composition according to claim 1 or aqueous emulsion or suspension according to claim 16, **characterised in that** at least one of the substituents $R_{10}$, $R'_{10}$ and $R'_{11}$ comprises a polyethylene glycol chain fragment of at least 5 ethylene oxide units.

**47.** Composition according to claim 1 or aqueous emulsion or suspension according to claim 16, **characterised in that** at least one of the substituents $R_{10}$, $R'_{10}$ and $R'_{11}$ comprises a polyethylene glycol chain fragment of at least 7 ethylene oxide units.

**48.** Composition according to claim 1 or aqueous emulsion or suspension according to claim 16 or compound according

to claim 34 or use according to claim 39, **characterised in that** E represents phosphorus.